# EUROPEAN PATENT APPLICATION

(11) **EP 4 524 146 A1**
(43) Date of publication of application: **19.03.2025**
(21) Application number: 23803589.3
(22) Date of filing: 10.05.2023
(51) Int. Cl.: C07H 19/167

(54) **MRNA CAP ANALOG AND METHOD FOR PRODUCING SAME, METHOD FOR PRODUCING MRNA, AND KIT**

(30) Priority: 10.05.2022 JP 2022077734
(71) Applicant: YAMASA CORPORATION, Choshi-shi, Chiba 288-0056 (JP)
(72) Inventor: KOHGO,Satoru, Choshi-shi, Chiba 288-0056 (JP)
(74) Representative: Maiwald GmbH
(86) International application number: PCT/JP2023/017621
(87) International publication number: WO 2023/219114

(57) **Abstract**

A mRNA cap analog includes a linker at a sugar or a base moiety of a nucleotide, wherein the linker includes: a functional group selected from an amino group, a carboxylic acid active ester group, an aminooxy group, a hydrazino group, a carbonyl group, a thiol group, a haloacetyl group, a Michael acceptor functional group, an alkynyl group, and a 1,3-dipole functional group; and a structure cleavable by light or a fluoride ion.

## Description

### [Technical Field]

The present invention relates to a mRNA cap analog and a method for producing the same, a method for producing mRNA, and a kit.

### [Background Art]

One of the known methods for producing mRNA (messenger RNA) is *in vitro* transcription. In this method, nucleoside triphosphate (NTP) is polymerized by RNA polymerase using a template DNA. In this reaction, a cap structure can be introduced at the 5' end of a mRNA (hereinafter referred to as "5' cap structure") by coexistence of a mRNA cap analog called ARCA (anti-reverse CAP analog) or the like. Patent Literature 1 discloses such a mRNA cap analog useful for *in vitro* transcription.

### [Citation List]

### [Patent Literature]

[PTL 1]
Japanese Patent Application Publication No. 2009-544754 A

### [Summary of Invention]

### [Technical Problem]

In the method for producing mRNA by *in vitro* transcription, the mRNA having the 5' cap structure can be produced, while mRNA having no 5' cap structure (i.e., having a triphosphate structure at the 5' end) is produced as a byproduct. The mRNA having no 5' cap structure is desirable to be separated and removed because it will induce spontaneous immunity and reduce a translation efficiency, but it is difficult to separate the mRNA by a purification method such as chromatography. Therefore, separation of the mRNA having no 5' cap structure produced as a by-product in the production of mRNA by *in vitro* transcription can be a useful approach for production of active pharmaceutical ingredients using mRNAs.

The present invention has been made to solve the above problems. An object of the present invention is to provide a mRNA cap analog and a method for producing the same, a method for producing a mRNA, and a kit, which can separate a mRNA having a 5' cap structure from a mRNA having no 5' cap structure.

### [Solution to Problem]

As a result of intensive studies for solving the above problems, the present inventors have found that mRNA having the 5' cap structure can be separated from mRNA having no 5' cap structure by introducing a linker having a specific structure into the sugar or base moiety of the nucleotide making up the mRNA cap analog, and they have completed the present invention. That is, the present invention is exemplified as follows:

[1] A mRNA cap analog, comprising a linker at a sugar or a base moiety of a nucleotide, the linker comprising a functional group selected from an amino group, a carboxylic acid active ester group, an aminooxy group, a hydrazino group, a carbonyl group, a thiol group, a haloacetyl group, a Michael acceptor functional group, an alkynyl group, and a 1,3-dipole functional group, the linker having a structure cleavable by light or a fluoride ion.
[2] The mRNA cap analog according to [1], wherein the linker has an o-nitrobenzyl ether structure or a silyl ether structure.
[3] The mRNA cap analog according to [1] or [2], wherein the mRNA cap analog is represented by the following general formula (1) or (2): 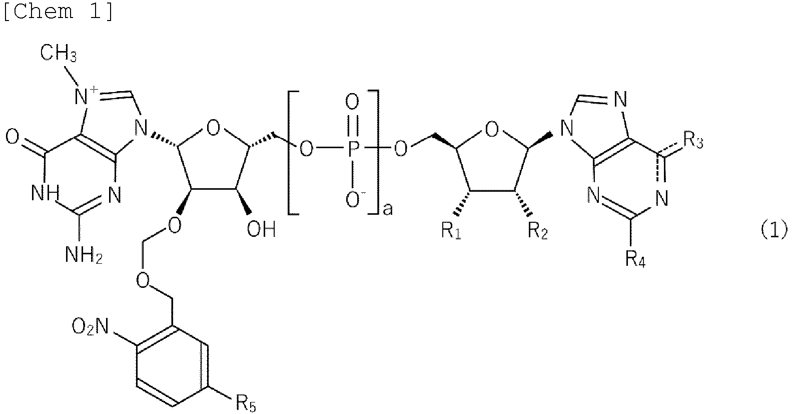 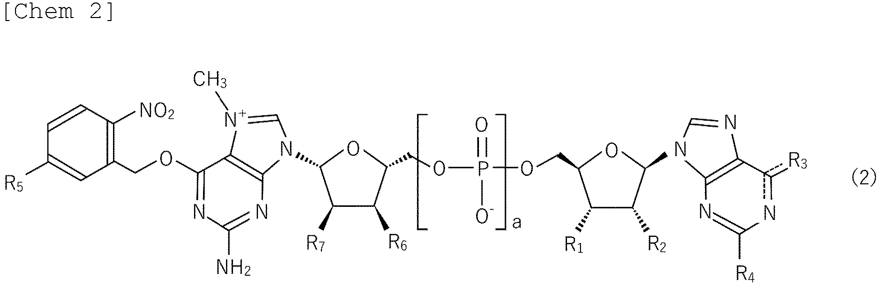 in which:
   R₁ is OH, OCH₃ or a group represented by the following general formula (3): 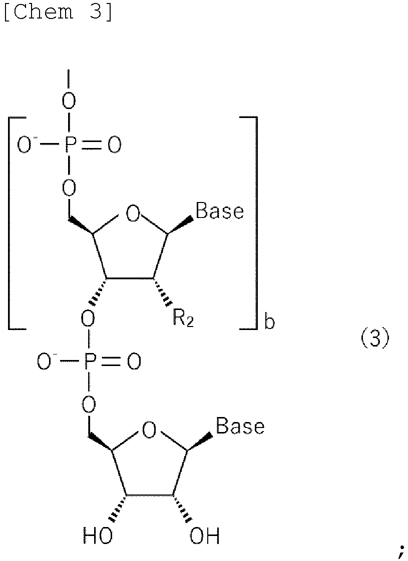
   R₂ is OH or OCH₃;
   R₃ is O, OH, NH₂, NHCH₃ or N(CH₃)₂, and when R₃ is O, then the bond between the C bonded to R₃ and R₃ is a double bond, and the bond between the C bonded to R₃ and N adjacent thereto is a single bond, and when R₃ is OH, NH₂, NHCH₃ or N(CH₃)₂, then the bond between the C bonded to R₃ and R₃ is a single bond, and the bond between the C bonded to R₃ and N adjacent thereto is a double bond;
   R₄ is NH₂, NHCH₃, N(CH₃)₂ or H;
   R₅ is a propargyloxy group or an azidoethoxy group;
   R₆ and R₇, which may be identical or different, are OH or OCH₃;
   a is 3 to 6;
   b is 0 to 19; and
   Base is adenine-9-yl, N6-methyladenine-9-yl, guanine-9-yl, cytosine-1-yl, 5-methylcytosine-1-yl, uracil-1-yl, uracil-5-yl or 1-methyluracil-5-yl.
[4] The mRNA cap analog according to [3], wherein R₃ is O and R₄ is NH₂.
[5] A method for producing a mRNA having a 5' cap structure, the method comprising the steps of:
   mixing a mRNA cap analog comprising a linker having a functional group A and a structure cleavable by light or fluoride ion at a sugar or a base moiety of a nucleotide with a nucleoside triphosphate, a template DNA and an RNA polymerase, and obtaining a preparation comprising a mRNA having a 5' cap structure by *in vitro* transcription;
   mixing the preparation with an insoluble support having a functional group B capable of reacting with the functional group A to generate a conjugate of the mRNA and the insoluble support;
   filtering and washing the conjugate; and
   applying light or a fluoride ion to the conjugate to cleave the linker and isolate the mRNA having the 5' cap structure.
[6] The method according to [5], wherein the functional groups A and B are a combination of an amino group and a carboxylic acid activated ester, a combination of an aminooxy group or a hydrazino group and a carbonyl group, a combination of a thiol group and a thiol group, a haloacetyl group or a Michael acceptor functional group, or a combination of an alkynyl group and a 1,3-dipole functional group.
[7] The method according to [5] or [6], wherein the linker has an o-nitrobenzyl ether structure or a silyl ether structure.
[8] The method according to [5], wherein the mRNA cap analog is represented by the following general formula (1) or (2): 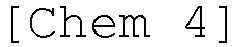 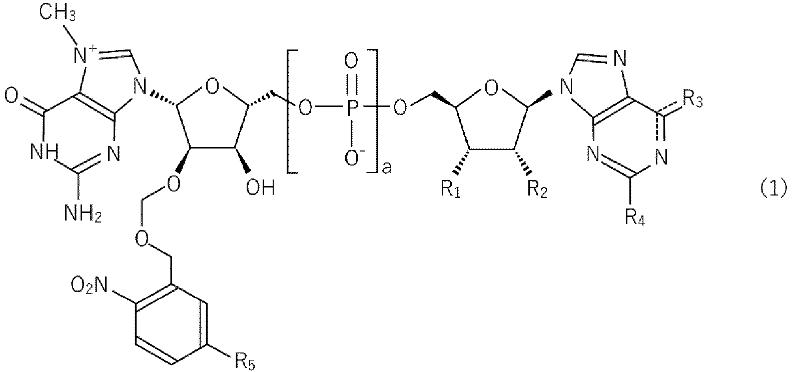 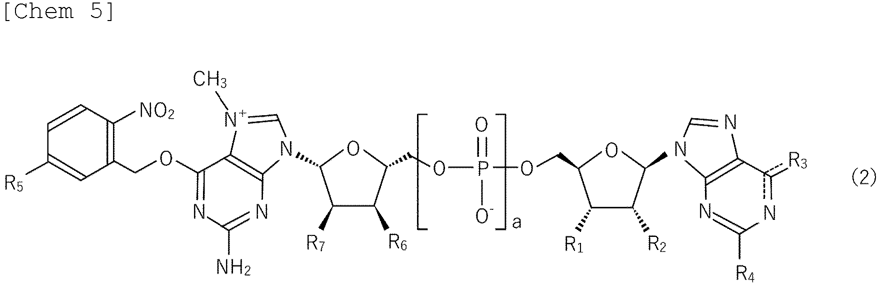 in which:
   R₁ is OH, OCH₃ or a group represented by the following general formula (3): 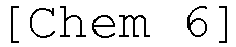 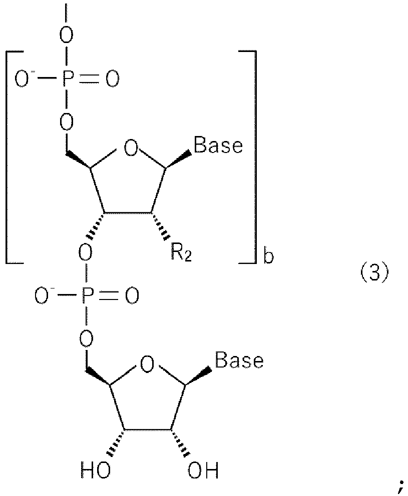
   R₂ is OH or OCH₃;
   R₃ is O, OH, NH₂, NHCH₃ or N(CH₃)₂, and when R₃ is O, then the bond between the C bonded to R₃ and R₃ is a double bond, and the bond between the C bonded to R₃ and N adjacent thereto is a single bond, and when R₃ is OH, NH₂, NHCH₃ or N(CH₃)₂, then the bond between the C bonded to R₃ and R₃ is a single bond, and the bond between the C bonded to R₃ and N adjacent thereto is a double bond;
   R₄ is NH₂, NHCH₃, N(CH₃)₂ or H;
   R₅ is a propargyloxy group or an azidoethoxy group;
   R₆ and R₇, which may be identical or different, are OH or OCH₃;
   a is 3 to 6;
   b is 0 to 19; and
   Base is adenine-9-yl, N6-methyladenine-9-yl, guanine-9-yl, cytosine-1-yl, 5-methylcytosine-1-yl, uracil-1-yl, uracil-5-yl or 1-methyluracil-5-yl.
[9] The method according to [8], wherein R₃ is O and R₄ is NH₂.
[10] The method according to [8] or [9], wherein, when R₅ is a propargyloxy group, then the insoluble support is represented by the following formula (4): 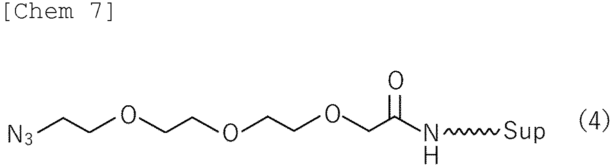 in which Sup is an insoluble support structural moiety.
[11] The method according to [8] or [9], wherein, when R₅ is an azidoethoxy group, the insoluble support is represented by the following formula (5), (6) or (7): 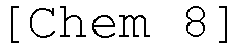 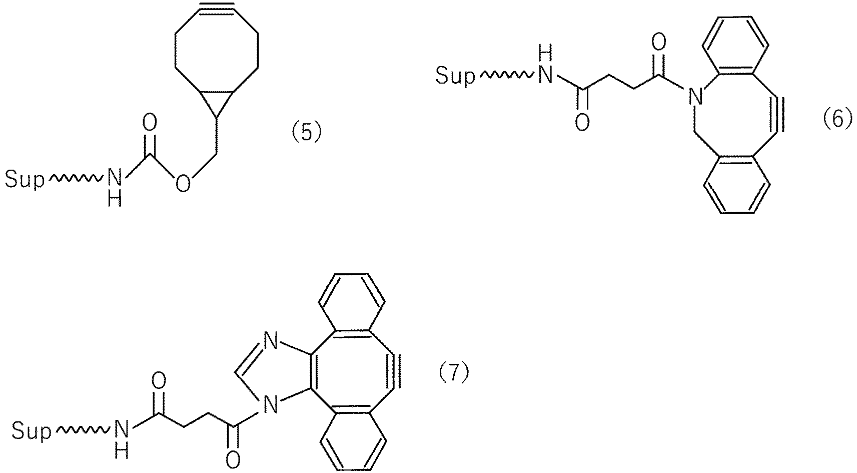 in which Sup is an insoluble support structural moiety.
[12] A method for producing a mRNA cap analog, the method comprising the steps of:
   introducing into a sugar moiety or a base moiety of a first nucleotide a linker comprising a functional group selected from an amino group, a carboxylic acid active ester group, an aminooxy group, a hydrazino group, a carbonyl group, a thiol group, a haloacetyl group, a Michael acceptor functional group, an alkynyl group, and a 1,3-dipole functional group, the linker having a structure cleavable by light or a fluoride ion; and
   linking the first nucleotide having the linker introduced therein to a second nucleotide in the presence of magnesium chloride.
[13] A kit for use in production of a mRNA having a 5' cap structure, the kit comprising:
   a mRNA cap analogue comprising a linker at a sugar moiety or a base moiety of a nucleotide, the linker having a functional group A selected from an amino group, a carboxylic acid active ester group, an aminooxy group, a hydrazino group, a carbonyl group, a thiol group, a haloacetyl group, a Michael acceptor functional group, an alkynyl group, and a 1,3-dipole functional group, the linker having a structure cleavable by light or a fluoride ion; and
   an insoluble support having a functional group B capable of reacting with the functional group A.
[14] The kit according to [13], wherein the functional groups A and B are a combination of an amino group and a carboxylic acid activated ester, a combination of an aminooxy group or a hydrazino group and a carbonyl group, a combination of a thiol group and a thiol group, a haloacetyl group or a Michael acceptor functional group, or a combination of an alkynyl group and a 1,3-dipole functional group.
[15] The kit according to [13] or [14], wherein the linker has an o-nitrobenzyl ether structure or a silyl ether structure.
[16] The kit according to [13], wherein the mRNA cap analog is represented by the following general formula (1) or (2): 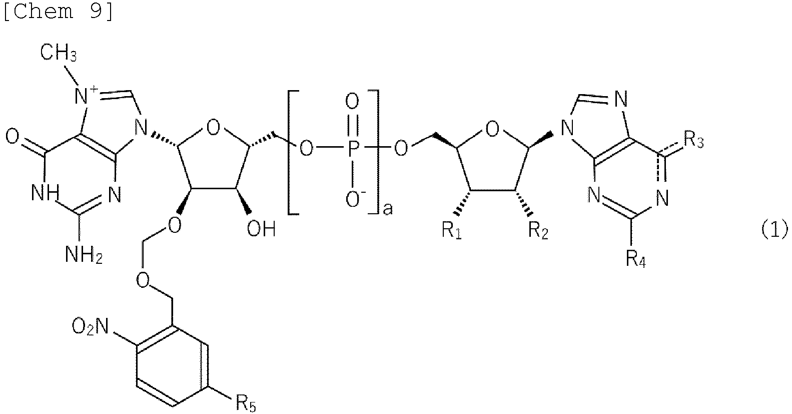 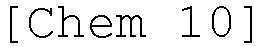 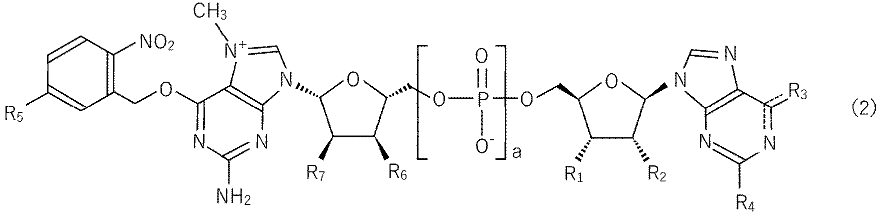 in which:
   R₁ is OH, OCH₃ or a group represented by the following general formula (3): 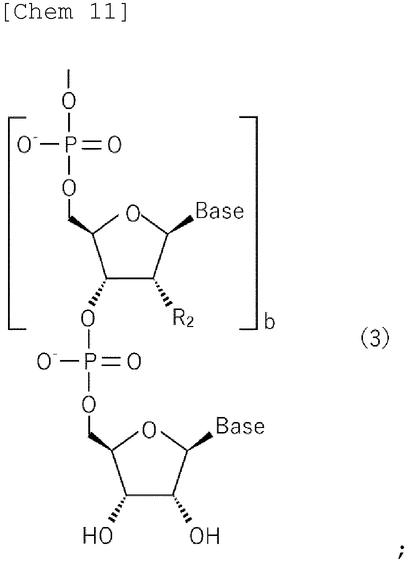
   R₂ is OH or OCH₃;
   R₃ is O, OH, NH₂, NHCH₃ or N(CH₃)₂, and when R₃ is O, then the bond between the C bonded to R₃ and R₃ is a double bond, and the bond between the C bonded to R₃ and N adjacent thereto is a single bond, and when R₃ is OH, NH₂, NHCH₃ or N(CH₃)₂, then the bond between the C bonded to R₃ and R₃ is a single bond, and the bond between the C bonded to R₃ and N adjacent thereto is a double bond;
   R₄ is NH₂, NHCH₃, N(CH₃)₂ or H;
   R₅ is a propargyloxy group or an azidoethoxy group;
   R₆ and R₇, which may be identical or different, are OH or OCH₃;
   a is 3 to 6;
   b is 0 to 19; and
   Base is adenine-9-yl, N6-methyladenine-9-yl, guanine-9-yl, cytosine-1-yl, 5-methylcytosine-1-yl, uracil-1-yl, uracil-5-yl or 1-methyluracil-5-yl.
[17] The kit according to [16], wherein R₃ is O and R₄ is NH₂.
[18] The kit according to [16] or [17], wherein, when R₅ is a propargyloxy group, then the insoluble support is represented by the following formula (4): 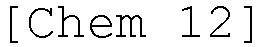 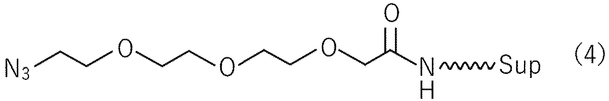 in which Sup is an insoluble support structural moiety.
[19] The kit according to [16] or [17], wherein, when R₅ is an azidoethoxy group, the insoluble support is represented by the following formula (5), (6) or (7): 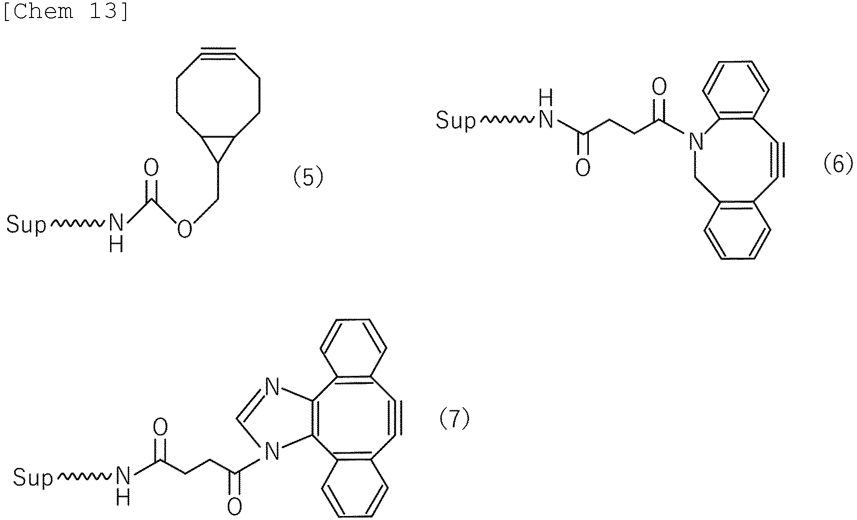 in which Sup is an insoluble support structural moiety.
[20] The kit according to any one of [13] to [19], further comprising one or more selected from nucleoside triphosphates, template DNAs, and RNA polymerases.

### [Advantageous Effects of Invention]

According to the present invention, it is possible to provide a mRNA cap analog and a method for producing the same, a method for producing mRNA, and a kit, which can separate a mRNA having a 5' cap structure from a mRNA having no 5' cap structure.

### [Brief Description of Drawings]

[FIG. 1]

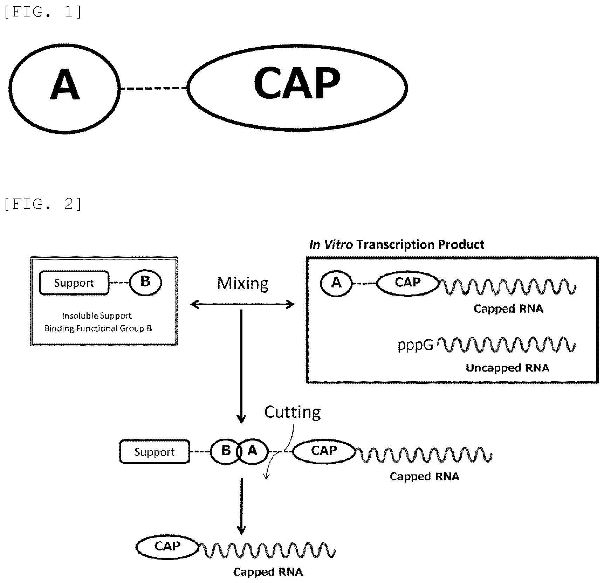

   FIG. 1 is a schematic view illustrating a structure of a mRNA cap analog according to the present invention.
[FIG. 2]
   FIG. 2 is a schematic view illustrating a process of separating a mRNA having a 5' cap structure from a mRNA having no 5' cap structure, which are produced by *in vitro* transcription using a mRNA cap analog according to the present invention.
[FIG. 3]

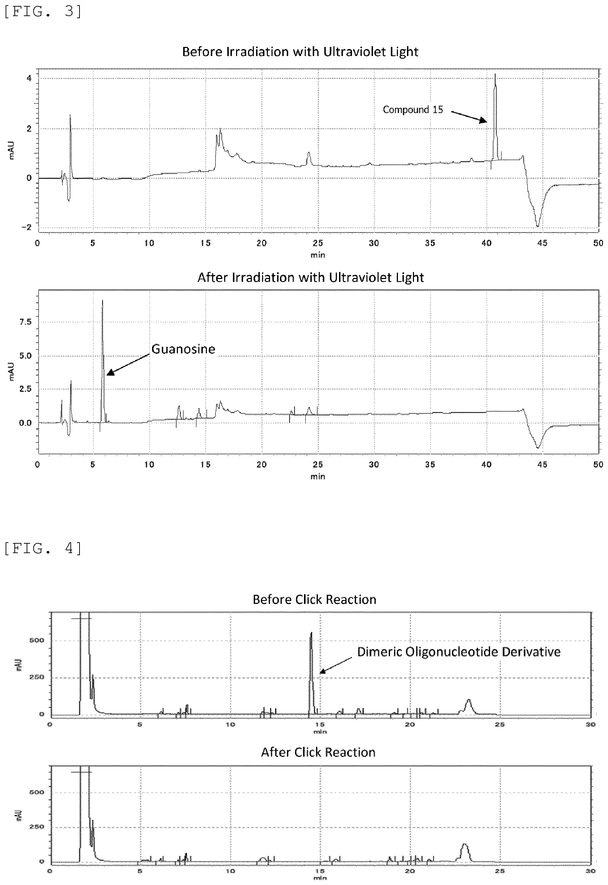

   FIG. 3 shows results of HPLC for an aqueous solution of Compound 15 before and after irradiation with ultraviolet light.
[FIG. 4]
   FIG. 4 shows results of HPLC for a solution of a dimeric oligonucleotide derivative before and after a click reaction.
[FIG. 5]

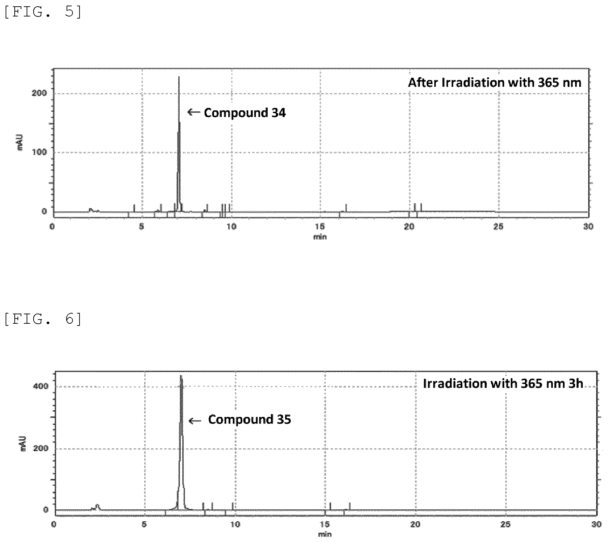

   FIG. 5 shows results of HPLC for a solution of a conjugate (Compound 32) after irradiation with ultraviolet light.
[FIG. 6]
   FIG. 6 shows results of HPLC for a solution of a conjugate (Compound 33) after irradiation with ultraviolet light.
[FIG. 7]

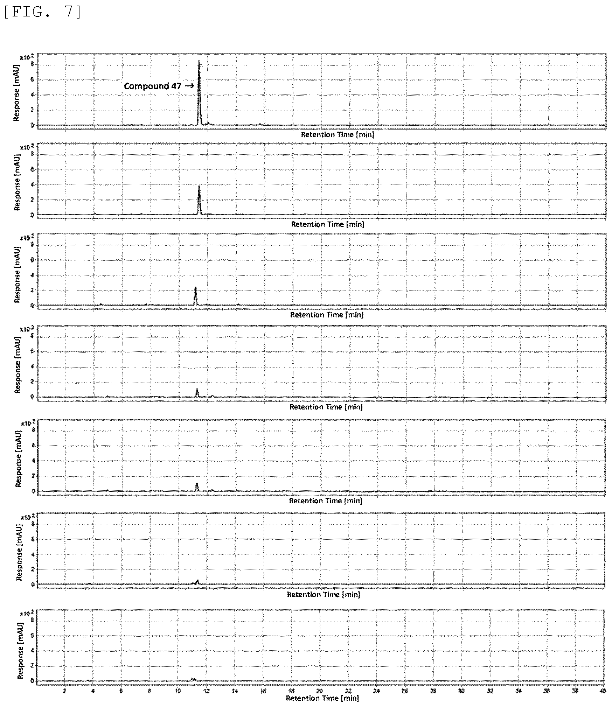

   FIG. 7 shows results of HPLC for a click reaction of Compound 47 with Compound 60.
[FIG. 8]

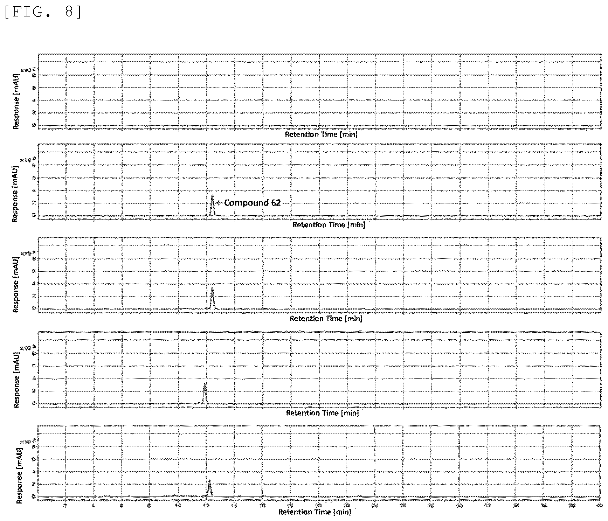

   FIG. 8 shows results of HPLC for Compound 61 upon irradiation with ultraviolet light.
[FIG. 9]

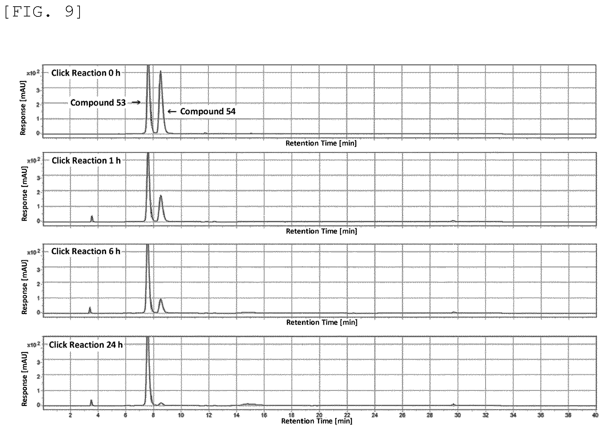

   FIG. 9 shows results of HPLC for a click reaction of Compound 54 with Compound 60.
[FIG. 10]

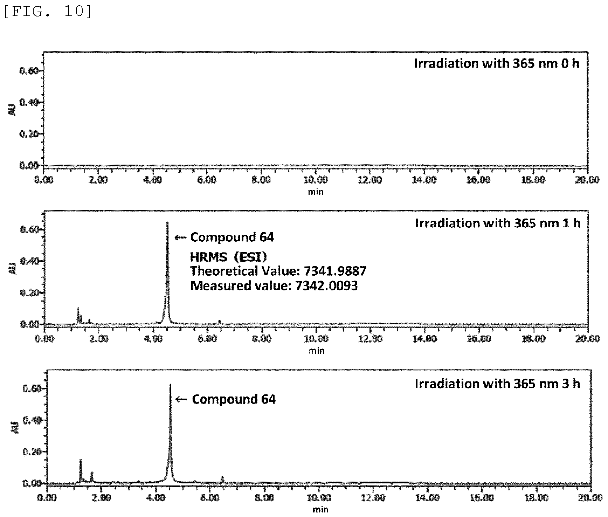

   FIG. 10 shows results of HPLC for Compound 63 upon irradiation with ultraviolet light.
[FIG. 11]

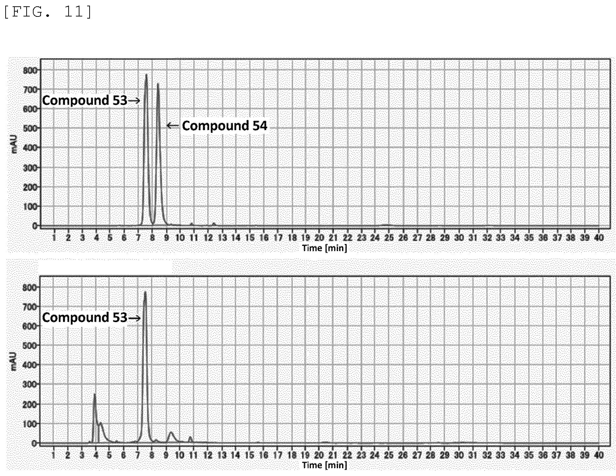

   FIG. 11 shows results of HPLC for a click reaction of Compound 54 with Compound 72.
[FIG. 12]

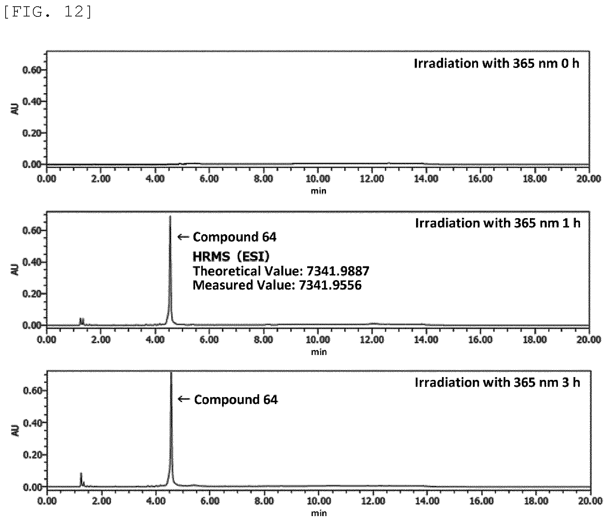

   FIG. 12 shows results of HPLC for Compound 73 upon irradiation with ultraviolet light.

### [Description of Embodiments]

The concept of the mRNA cap analog according to the present invention will be described with reference to FIGS. 1 and 2.

FIG. 1 is a schematic view for explaining the structure of the mRNA cap analog according to the present invention.

As shown in FIG. 1, the mRNA cap analog according to the present invention has: a nucleotide (represented as "CAP") that provides a 5' cap structure; and a functional group A (represented as "A") capable of reacting with a functional group B, in which they are linked via a structure (shown by a dotted line) cleavable by light or a fluoride ion. As used herein, the moiety represented by A and the dotted line in FIG. 1 is referred to as a "linker".

FIG. 2 is a schematic view for explaining a process of separating a mRNA having a 5' cap structure from a mRNA having no 5' cap structure, which are produced by *in vitro* transcription using the mRNA cap analog according to the present invention.

As shown in FIG. 2, the mRNA having the 5' cap structure (referred to as "Capped RNA") and the mRNA having no 5' cap structure (referred to as "Uncapped RNA") are produced by *in vitro* transcription. Since the mRNA having the 5' cap structure has the functional group A capable of reacting with a functional group B (referred to as "B"), the functional group A being introduced into the 5' cap structure, it is allowed to react with an insoluble support having the functional group B to produce a conjugate, so that only the mRNA having no 5' cap structure can be separated and removed by filtration and washing. The linker of the separated and recovered conjugate is cleaved (cut) by application of light or a fluoride ion, so that only the mRNA having the 5' cap structure is isolated.

Hereinafter, embodiments of the present invention will be specifically described. It is to understand that the present invention is not limited to the following embodiments, and those which appropriately added changes, improvements and the like to the following embodiments based on knowledge of a person skilled in the art without departing from the spirit of the present invention fall within the scope of the present invention.

### <mRNA Cap Analog>

The mRNA cap analog according to the embodiment of the present invention includes a linker at a sugar moiety or a base moiety of a nucleotide, the linker having: a functional group A selected from an amino group, a carboxylic acid active ester group, an aminooxy group, a hydrazino group, a carbonyl group, a thiol group, a haloacetyl group, a Michael acceptor functional group, an alkynyl group, and a 1,3-dipole functional group; and a structure cleavable by light or a fluoride ion. The mRNA cap analog having such a structure can be bound to an insoluble support having a functional group B capable of reacting with the functional group A by various known reactions, so that the mRNA having the 5' cap structure can be separated from the mRNA having no 5' cap structure.

As used herein, the term "mRNA cap analog" refers to a capping reagent used for producing the mRNA having the 5' cap structure by *in vitro* transcription.

As used herein, the "nucleotide" is a compound including a base and a sugar having a phosphate group covalently bonded thereto. The nucleotide may be modified with any of a variety of substituents.

When the functional group A of the mRNA cap analog is the amino group, the combination with the insoluble support in which the functional group B is the carboxylic acid active ester group can lead to production of a conjugate having an amide bond (-CONH-) generated by a reaction of the mRNA having the 5' cap structure having the introduced functional group A with the insoluble support having the functional group B. Similarly, when the functional group A of the mRNA cap analog is the carboxylic acid active ester group, the combination with the insoluble support in which the functional group B is the amino group can lead to production of a conjugate having an amide bond generated by a reaction of the mRNA having the 5' cap structure having the introduced functional group A with the insoluble support having the functional group B.

Non-limiting examples of the amino group include a monomethylamino group, a monoethylamino group, a mono-n-propylamino group, a monoisopropylamino group, a mono-n-butylamino group, a mono-sec-butylamino group, a mono-tert-butylamino group, a mono-isobutylamino group, a tert-butylisopropylamino group, a mono-n-hexylamino group, a mono-n-octylamino group, a mono-n-decylamino group, a monophenylamino group, a trimethylsilylamino group, a mono-tert-butyldimethylsilylamino group, a pyrrolidinyl group, a piperidinyl group, a carbazolyl group, a dihydroindolyl group, a dihydroisoindolyl group, and so on. The amino group may be optionally substituted.

Non-limiting examples of the carboxylic acid active ester group include halides, thiophenyl esters, 4-nitrophenyl thioesters, cyanomethyl esters, nitro esters, dinitrophenyl esters, N-hydroxysuccinimide esters, p-nitrophenyl esters, 2,3,5-trichlorophenyl esters, 2,4,6-trichlorophenyl esters, 2,4,5-trichlorophenyl esters, pentachlorophenyl esters, 2,4-dinitrophenyl esters, and N-hydroxyphthalimide esters of carboxylic acids, and so on.

When the functional group A of the mRNA cap analog is the aminooxy group, the combination with the insoluble support in which the functional group B is the carbonyl group can lead to production of a conjugate having an oxime bond (-CR=N- in which R is H or alkyl) generated by the reaction of the mRNA having the 5' cap structure having the introduced functional group A with the insoluble support having the functional group B. Similarly, when the functional group A of the mRNA cap analog is the carbonyl group, the combination with the insoluble support in which the functional group B is the aminooxy group can lead to production of a conjugate having an oxime bond generated by the reaction of the mRNA having the 5' cap structure having the introduced functional group A with the insoluble support having the functional group B.

Non-limiting examples of the carbonyl group include a methoxycarbonyl group, a tert-butoxycarbonyl group, an ethoxycarbonyl group, an aldehyde group, a ketone group, and so on. The carbonyl group may be optionally substituted.

When the functional group A of the mRNA cap analog is the hydrazino group, the combination with the insoluble support in which the functional group B is the carbonyl group can lead to production of a conjugate having a hydrazone bond (-CR=N-NHR- in which R is H or alkyl) generated by the reaction of the mRNA having the 5' cap structure having the introduced functional group A with the insoluble support having functional group B. Similarly, when the functional group A of the mRNA cap analog is the carbonyl group, the combination with the insoluble support in which the functional group B is the hydrazino group can lead to production of a conjugate having a hydrazone bond generated by the reaction of the mRNA having the 5' cap structure having the introduced functional group A with the insoluble support having the functional group B.

Non-limiting examples of the hydrazino group include a methylhydrazino group, an ethylhydrazino group, and so on. The hydrazino group may be optionally substituted.

When the functional group A of the mRNA cap analog is the thiol group, the combination with the insoluble support in which the functional group B is the thiol group can lead to production of a conjugate having a disulfide bond (-SS-) generated by the reaction of the mRNA having the 5' cap structure having the introduced functional group A with the insoluble support having the functional group B.

When the functional group A of the mRNA cap analog is the haloacetyl group, the combination with the insoluble support in which the functional group B is the thiol group can lead to production of a conjugate having a thioether bond (-S-) generated by the reaction of the mRNA having the 5' cap structure having the introduced functional group A with the insoluble support having the functional group B. Similarly, when the functional group A of the mRNA cap analog is the thiol group, the combination with the insoluble support in which the functional group B is the haloacetyl group can lead to production of a conjugate having a thioether bond generated by the reaction of the mRNA having the 5' cap structure having the introduced functional group A with the insoluble support having the functional group B.

Non-limiting examples of the haloacetyl group include a chloroacetyl group, a bromoacetyl group, an iodoacetyl group and so on. The haloacetyl group may be optionally substituted.

When the functional group A of the mRNA cap analog is the Michael acceptor functional group, the combination with the insoluble support in which the functional group B is the thiol group can lead to production of a conjugate that is a Michael adduct generated by the reaction of the mRNA having the 5' cap structure having the introduced functional group A with the insoluble support having the functional group B. Similarly, when the functional group A of the mRNA cap analog is the thiol group, the combination with the insoluble support in which the functional group B is the Michael acceptor functional group can lead to production of a conjugate that is a Michael adduct of the mRNA having the 5' cap structure having the introduced functional group A with the insoluble support having the functional group B.

As used herein, the term "Michael acceptor functional group" refers to a functional group that can be a Michael acceptor. Non-limiting examples of the Michael acceptor functional group include a maleimide group, an α,β-unsaturated carbonyl group, and so on. The Michael acceptor functional group may be optionally substituted.

When the functional group A of the mRNA cap analog is the alkynyl group, the combination with the insoluble support in which the functional group B is the 1,3-dipolar functional group can lead to production of a conjugate having a bond due to 1,3-dipolar cyclization generated by the reaction of the mRNA having the 5' cap structure having the introduced functional group A with the insoluble support having the functional group B. Similarly, when the functional group A of the mRNA cap analog is the 1,3-dipolar functional group, the combination with the insoluble support in which the functional group B is the alkynyl group can lead to production of a conjugate having a bond due to 1,3-dipolar cyclization generated by the reaction of the mRNA having the 5' cap structure having the introduced functional group A with the insoluble support having the functional group B. The reaction generated by this combination is called a click reaction. The click reaction is particularly preferable among the above various combinations because it can perform a quantitative reaction under very mild conditions.

The alkynyl group is a linear or branched hydrocarbon chain containing one or more triple bonds. The alkynyl group can have, for example, from 2 to 20 carbon atoms. Typical alkynyl groups include ethynyl, propynyl, butynyl, pentynyl, and hexynyl groups, and the like. The alkynyl group may be optionally substituted.

Non-limiting examples of the 1,3-dipole functional group include a nitrone group, an azomethine imine group, an azomethine ylide group, an azimine group, an azoxy group, a nitro group, a carbonyl ylide group, a carbonyl imine group, a carbonyl oxide group, a nitrosoimine group, a nitrosoxide group, a nitrile oxide group, a nitrile imine group, a nitrile ylide group, a diazoalkane group, and an azido group. These may be optionally substituted. For example, when the 1,3-dipole functional group is the azido group, a triazole ring can be formed by bonding with the alkynyl group by the click reaction.

Non-limiting examples of the linker structure cleavable by light or a fluoride ion include an o-nitrobenzyl ether structure or a silyl ether structure.

The o-nitrobenzyl ether structure can be cleaved (cut) by application of light (typically, irradiation with ultraviolet light having a wavelength of 365 nm).

The silyl ether structure can be cleaved (cut) by the action of the fluoride ion. Tetrabutylammonium fluoride (TBAF), cesium fluoride (CsF) and the like can be used as a source of the fluoride ion.

The base moiety of the nucleotide to which the linker is linked is not particularly limited and may be adenine (A), guanine (G), cytosine (C) or uracil (U), the guanine (G) being preferred.

The position of the sugar moiety of the nucleotide to which the linker is linked is not particularly limited and may be the carbon at the 2'-portion or the carbon at the 3'-position, but it is preferably the carbon at the 2'-position.

The position of the base moiety of the nucleotide to which the linker is linked is not particularly limited, but in the case of adenine or guanine having a purine skeleton, it is preferably the carbon at the 2-position or the carbon at the 6-position, and in the case of cytosine or uracil having a pyrimidine skeleton, it is preferably the nitrogen at the 3-position, the carbon at the 4-position, the carbon at the 5-position, or the carbon at the 6-position.

The mRNA cap analogue according to the embodiment of the present invention can be typically represented by the following general formula (1) or (2). The mRNA cap analogue having such a structure also has a good efficiency for *in vitro* transcription reaction.

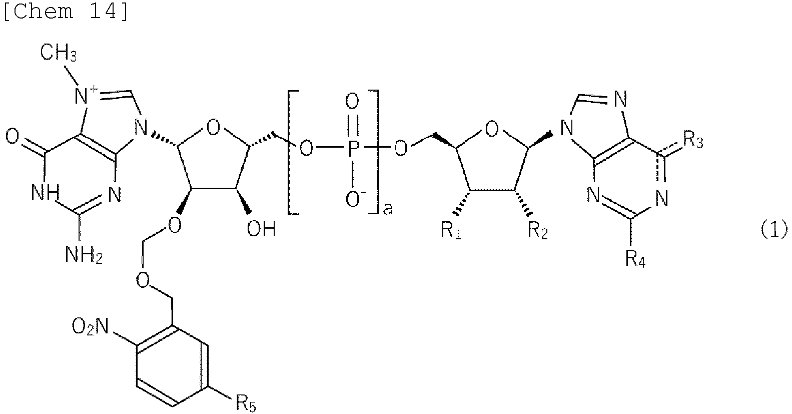

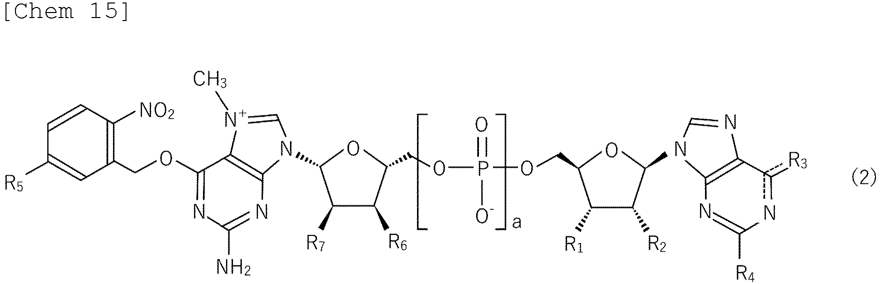

In the above formulae, R₁ is OH, OCH₃ or a group represented by the following general formula (3):

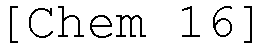

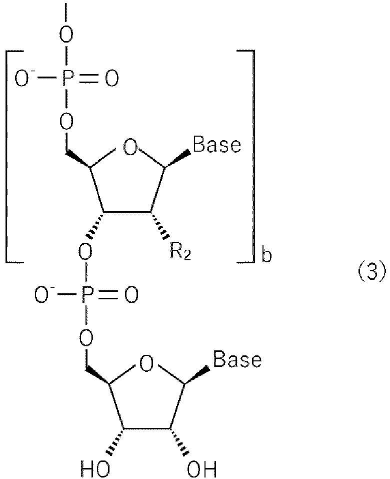

In the above formulae, R₂ is OH or OCH₃.

In the above formulae, R₃ is O, OH, NH₂, NHCH₃ or N(CH₃)₂, and when R₃ is O, the bond between the C bonded to R₃ and R₃ (the bond represented by the dotted line and the solid line) is a double bond, and the bond between the C bonded to R₃ and N adjacent thereto (the bond represented by the dotted line and the solid line) is a single bond, and when R₃ is OH, NH₂, NHCH₃ or N(CH₃)₂, the bond between the C bonded to R₃ and R₃ (the bond represented by the dotted line and the solid line) is a single bond, and the bond between the C bonded to R₃ and the N adjacent thereto (the bond represented by the dotted line and the solid line) is a double bond. It is preferable that R₃ is O.

In the above formulae, R₄ is NH₂, NHCH₃, N(CH₃)₂ or H.

It is preferable that R₄ is NH₂.

In the above formulae, R₅ is a propargyloxy group or an azidoethoxy group. It is preferable that R₅ is the azidoethoxy group.

In the above formula, R₆ and R₇, which may be identical or different, are OH or OCH₃.

In the above formulae, a is 3 to 6. It is preferable that a is 3.

In the above formulae, b is 0 to 19.

In the above formula, the Base is adenine-9-yl, N6-methyladenine-9-yl, guanine-9-yl, cytosine-1-yl, 5-methylcytosine-1-yl, uracil-1-yl, uracil-5-yl or 1-methyluracil-5-yl.

The mRNA cap analog according to the embodiment of the present invention can be produced according to a known method. An example of the method for producing the mRNA cap analog according to the embodiment of the present invention is described below.

The method for producing the mRNA cap analog according to the embodiment of the present invention includes the steps of: introducing a linker having a structure cleavable by light or a fluoride ion and a functional group A selected from an amino group, a carboxylic acid active ester group, an aminooxy group, a hydrazino group, a carbonyl group, a thiol group, a haloacetyl group, a Michael acceptor functional group, an alkynyl group, and a 1,3-dipole functional group, into a sugar moiety or a base moiety of a first nucleotide; and linking the first nucleotide having the introduced linker to a second nucleotide in the presence of magnesium chloride. The production method including such steps allows the mRNA cap analog to be efficiently produced.

The linker can be synthesized by using a known method depending on the types of the functional group A and the structure cleavable by light or a fluoride ion.

The conditions for introducing the linker into the sugar moiety or the base moiety of the first nucleotide are not particularly limited, and they may be appropriately set depending on the types of the linker and the first nucleotide. Non-limiting examples of the first nucleotide that can be used herein include guanylic acid, and so on.

The conditions for linking the first nucleotide having the introduced linker to the second nucleotide are not particularly limited, with the exception that the linking is performed in the presence of magnesium chloride, and they may be appropriately set depending on the types of the linker, the first nucleotide, and the second nucleotide. Non-limiting examples of the second nucleotide that can be used herein include guanosine diphosphate, and so on.

### <Method for Producing mRNA Having 5' Cap Structure>

The method for producing the mRNA having the 5' cap structure according to an embodiment of the present invention (which may, hereinafter, be abbreviated as a "production method of mRNA") includes a preparing step (hereinafter referred to as a "first step") for a preparation containing the mRNA having the 5' cap structure; a producing step of a conjugate (hereinafter referred to as a "second step"); a filtrating and washing step (hereinafter referred to as a "third step") for a conjugate; and an isolating step (hereinafter referred to as a "fourth step") of the mRNA having the 5' cap structure. The production method including such steps allows the mRNA having no 5' cap structure that is by-produced in the production of mRNA by *in vitro* transcription to be separated and removed to isolate the mRNA having the 5' cap structure, so that the production method is useful for use in a pharmaceutical drug substance.

The first step is to mix the mRNA cap analog having the linker at the sugar moiety or the base moiety of the nucleotide, which contains the functional group A and has the structure cleavable by light or the fluoride ion, with a nucleoside triphosphate, a template DNA, and an RNA polymerase, and to obtain a preparation containing the mRNA having the 5' cap structure by *in vitro* transcription. The preparation obtained in this step also includes the mRNA having no 5' cap structure.

The first step can be carried out according to existing conditions, with exception that the mRNA cap analog having the specific structure is used.

It should be noted that the details for the mRNA cap analog having the specific structure have already been described above, so their descriptions will be omitted.

The second step is to mix the insoluble support having the functional group B capable of reacting with the functional group A with the above preparation to generate a conjugate of the mRNA having the 5' cap structure and the insoluble support.

The conditions for generating the conjugate are not particularly limited and they may be set according to the types of the functional group A of the mRNA having the 5' cap structure and the functional group B of the insoluble support.

The functional groups A and B may be a combination of an amino group and a carboxylic acid active ester, a combination of an aminooxy group or a hydrazino group and a carbonyl group, a combination of a thiol group and a thiol group, a haloacetyl group or a Michael acceptor functional group, or a combination of an alkynyl group and a 1,3-dipole functional group. As used herein, for example, the combination of the amino group and the carboxylic acid active ester means both a combination in which the functional group A is the amino group and the functional group B is the carboxylic acid active ester, and a combination in which the functional group A is the carboxylic acid active ester and the functional group B is an amino group. The same is true for the other combinations. By using such combinations, various bonds as described above can be formed. Among these combinations, the combination of the alkynyl group and the 1,3-dipole functional group, which is capable of carrying out the click reaction under simple conditions, is preferred.

The insoluble support is not particularly limited as long as it is insoluble in an aqueous solution. Examples of the insoluble support include supports made of polysaccharides such as agarose, alginate (alginic acid salt), carrageenan, chitin, cellulose, dextrin, dextran, and starch; supports made of synthetic polymers such as polyvinyl alcohol, polymethacrylate, poly(2-hydroxyethyl methacrylate), and polyurethane; and supports made of ceramics such as silica. Various commercially available insoluble supports may also be used. Examples of commercially available supports include Primer Support^{™} 5G (manufactured by Cytiva) and EAH-Sepharose^{™} 4B (manufactured by Cytiva), which are used in oligonucleotide synthesis.

When the mRNA cap analogue represented by the above general formula (1) or (2) in which R₅ is the propargyloxy group is used, the insoluble support can be represented by the following formula (4):

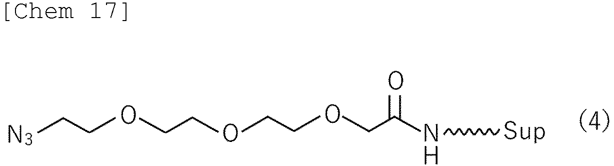

In formula (4), Sup is an insoluble support structural moiety. The insoluble support structural moiety is a moiety composed of the support as described above.

When the mRNA cap analogue represented by the above general formula (1) or (2) in which R₅ is the azidoethoxy group is used, the insoluble support can be represented by the following formula (5), (6), or (7). Such an insoluble support having the cyclooctyne group allows the click reaction to be carried out even in the absence of copper (I) ions, so that contamination with copper (I) ions during the production of mRNA can be avoided.

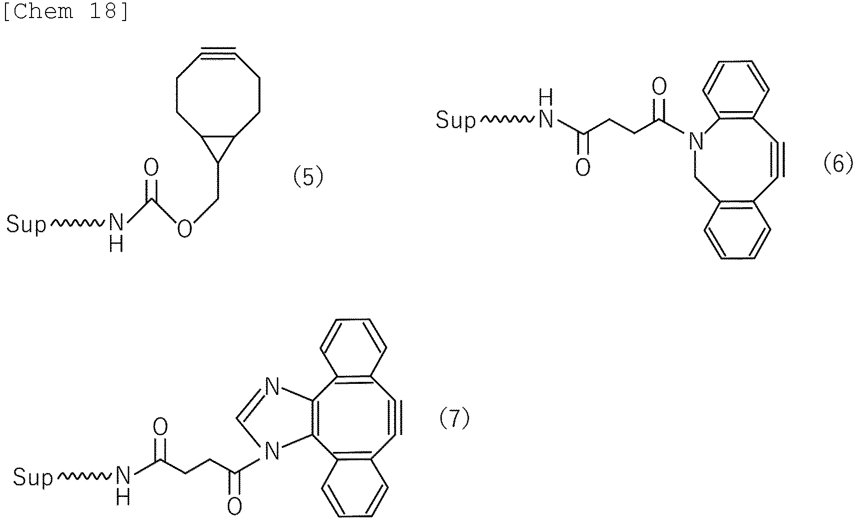

In the formula (5), (6) or (7), Sup is an insoluble support structural moiety. The insoluble support structural moiety is a moiety composed of the support as described above.

The third step is to filter and wash the conjugate. The conjugate of the mRNA having the 5' cap structure and the insoluble support is in a form of a solid, whereas the mRNA having no 5' cap structure is in a form of a liquid. Therefore, by filtering and washing the conjugate, the mRNA having no 5' cap structure can be separated and removed.

The method and conditions for filtering and washing are not particularly limited, and they can be performed in accordance with known methods.

The fourth step is to cleave the linker by applying light or the fluoride ion to the conjugate as described above, and to isolate the mRNA having the 5' cap structure. The cleavage of the linker using an acid or alkali may affect the mRNA having the 5' cap structure, but since the linker is cleaved with light or the fluoride ion, the effect on the mRNA having the 5' cap structure can be reduced.

The linker cleavage conditions are not particularly limited, and they may be appropriately set depending on the structure of the linker cleavable by light or the fluoride ion. For example, when the structure is the o-nitrobenzyl ether structure, the linker can be cleaved (cut) by irradiating the compound with ultraviolet light having a wavelength of 365 nm. When the structure is the silyl ether structure, the linker can be cleaved (cut) by applying a solution containing the fluoride ions to the compound.

### <Kit>

The kit according to the embodiment of the present invention is used for producing the mRNA having the 5' cap structure.

The kit according to the embodiment of the present invention includes: a mRNA cap analog having a linker at a sugar moiety or a base moiety of a nucleotide, the linker having: a functional group A selected from an amino group, a carboxylic acid active ester group, an aminooxy group, a hydrazino group, a carbonyl group, a thiol group, a haloacetyl group, a Michael acceptor functional group, an alkynyl group, and a 1,3-dipole functional group, the linker having a structure cleavable by light or a fluoride ion; and an insoluble support having a functional group B capable of reacting with the functional group A. The kit including such mRNA cap analog and insoluble support allows the mRNA having no 5' cap structure that is by-produced in the production of the mRNA having the 5' cap structure to be separated and removed to isolate the mRNA having the 5' cap structure.

The details for the mRNA cap analog and the insoluble support used in the kit according to the embodiment of the present invention have already been described above, so descriptions thereof will be omitted.

The kit according to the embodiment of the present invention may further include one or more components selected from nucleoside triphosphate, a template DNA, and RNA polymerase, as needed. These components are raw materials used in preparing the mRNA having the 5' cap structure by *in vitro* transcription. Therefore, the kit including these components allows the mRNA having the 5' cap structure to be easily prepared.

### [Examples]

While the present invention will be described below with reference to Examples, the present invention is not limited to these Examples in any way. In Examples, Me represents a methyl group, Et represents an ethyl group, and Ac represents an acetyl group.

### (1. Model Experiment on Cleavage of Linker)

In order to conduct model experiments on cleavage of linkers, nucleoside derivatives having various introduced linkers were synthesized and cleavage experiments were conducted.

Using 2-nitro-5-hydroxybenzaldehyde as a starting material, the three types of linkers having a methoxy group, a propargyloxy group, and a 2-azidoethoxy group as the functional groups A were synthesized, respectively. The nucleoside derivative was synthesized by introducing each of these linkers into the base moiety of guanosine.

### <Synthesis of Linker Having Methoxy Group as Functional Group A [2-nitro-5-methoxybenzyl alcohol (Compound 3)]>

First, 2-nitro-5-methoxybenzyl alcohol (Compound 3) was synthesized according to the following scheme:

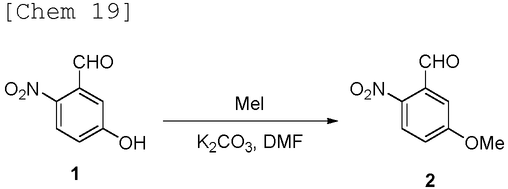

Compound 1 (200 mg, 1.20 mmol) was dissolved in N,N-dimethylformamide (3 mL), and methyl iodide (149 µL, 2.39 mmol) and potassium carbonate (662 mg, 4.79 mmol) were added, and stirred at room temperature for 16 hours. The reaction solution was diluted with ethyl acetate, and then washed twice with deionized water, and the organic layer was dried over anhydrous sodium sulfate and concentrated. The concentrate was purified by medium pressure silica gel column chromatography (hexane:ethyl acetate = 3:1) to obtain Compound 2 (157 mg, 867 µmol, 72.4%). ¹H-NMR (CDCl₃): δ 10.49 (s, 1H), 8.17 (d, 1H), 7.34 (d, 1H), 7.16 (dd, 1H), 3.96 (s, 3H)

Compound 3 was then synthesized according to the following scheme:

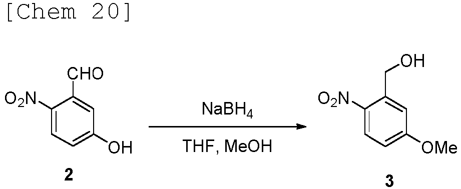

Compound 2 (157 mg, 867 µmol) was dissolved in tetrahydrofuran (2 mL), and methanol (4.3 mL) was added and cooled on ice. Sodium borohydride (16.4 mg, 433 µmol) was then added and stirred under the same conditions for 1 hour. The reaction solution was diluted with ethyl acetate and washed with water, and the organic layer was then dried over anhydrous sodium sulfate and concentrated. The concentrate was purified by medium pressure silica gel column chromatography (hexane:ethyl acetate = 3:1) to obtain Compound 3 (157 mg, 857 µmol, 98.9%).
¹H-NMR (CDCl₃): δ 8.19 (d, 1H), 7.22 (d, 1H), 6.90 (dd, 1H), 5.00 (d, 2H) 3.92 (s, 3H), 2.57 (t, 1H)

### <Synthesis of Linker Having Propargyloxy Group as Functional Group A [2-nitro-5-propargyloxybenzyl alcohol (Compound 5)]>

According to the following scheme, 2-nitro-5-propargyloxybenzyl alcohol (Compound 5) was synthesized:

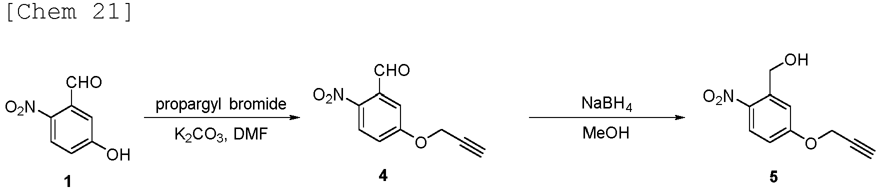

Compound 1 (3.00 g, 18.0 mmol) was dissolved in N,N-dimethylformamide (45.0 mL), and propargyl bromide (1.40 mL, 18.0 mmol) and potassium carbonate (5.0 g, 36 mmol) were added, and stirred at room temperature for 5 hours. The reaction solution was diluted with ethyl acetate, and then washed twice with deionized water, and the organic layer was then dried over anhydrous sodium sulfate and concentrated to obtain crude Compound 4.

The crude compound 4 was then dissolved in tetrahydrofuran (18.0 mL), and methanol (90 mL) was added, and cooled on ice. Sodium borohydride (0.34 g, 9.0 mmol) was added, and stirred for 20 minutes under the same conditions. The reaction solution was concentrated, and then diluted with ethyl acetate, and washed with water. The organic layer was dried over anhydrous sodium sulfate and concentrated, and the concentrate was crystallized from hexane-ethyl acetate. The crystals were collected by filtration, washed, and then dried in vacuum to obtain Compound 5 (3.32 g, 16.0 mmol, 89.0%).
¹H-NMR (CDCl₃): δ 8.20 (d, 1H), 7.33 (d, 1H), 7.00 (dd, 1H), 5.01 (d, 1H), 4.82 (d, 1H), 2.59 (t, 1H), 2.54 (t, 1H)

### <Synthesis of Linker Having 2-Azidoethoxy Group as Functional Group A [(5-(2-azidoethoxy)-2-nitrophenyl)methanol (Compound 12)]>

First, methyl 2-(3-formyl-4-phenoxy)acetate (Compound 6) was synthesized according to the following scheme:

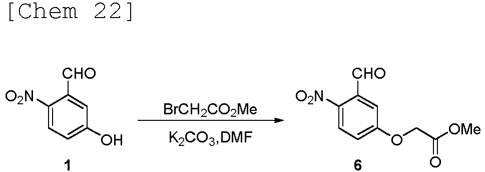

Compound 1 (10.0 g, 59.8 mmol) was dissolved in N,N-dimethylformamide (120 mL), and potassium carbonate (13.7 g, 120 mmol) and methyl bromoacetate (8.27 mL, 89.8 mmol) were then added in this order and stirred for 1 hour. At the end of the reaction, the mixture was diluted with ethyl acetate, and the organic layer was washed twice with water (120 mL). The aqueous layer was extracted with ethyl acetate (120 mL), and the organic layer were combined. The organic layer was then dried over magnesium sulfate, and the solvent was removed under reduced pressure, and hexane was added to the residue. The resulting solid was filtered, washed with hexane, and dried to obtain Compound 6 (12.9 g, 90.1%).
¹H-NMR (CDCl₃): δ 10.45 (s, 1H), 8.16 (d, 1H), 7.29 (d, 1H), 7.19 (dd, 1H), 4.79 (s, 2H), 3.82 (s, 3H)

Subsequently, Compound 7 was synthesized according to the following scheme:

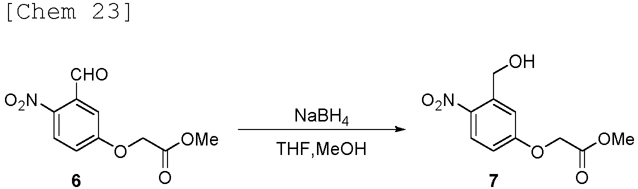

Compound 6 (12.7 g, 53.1 mmol) was dissolved in a mixed solvent of tetrahydrofuran and methanol (1:1, 106 mL), and sodium borohydride (703 mg, 18.6 mmol) was then added on ice and stirred for 1 hour. At the end of the reaction, the reaction solution was quenched with acetone (5 mL) and then concentrated. The concentrate was dissolved in ethyl acetate, and the organic layer was washed with a saturated aqueous ammonium chloride solution. The organic layer was then dried over magnesium sulfate and the solvent was distilled off under reduced pressure, and hexane was then added to the residue. The resulting solid was filtered, washed with hexane, and dried to obtain Compound 7 (11.31 g, 88.3%).
1H-NMR (DMSO-d6): δ 8.12 (d, 1H), 7.33 (d, 1H), 7.04 (dd, 1H), 5.61 (t, 1H), 4.99 (s, 2H), 4.84 (d, 2H), 3.72 (s, 3H)

Subsequently, Compound 8 was synthesized according to the following scheme:

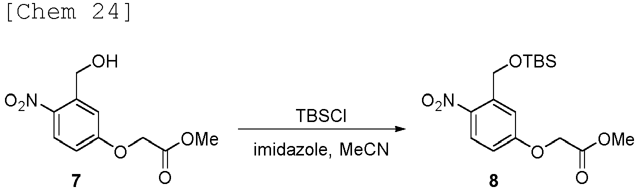

Compound 7 (11.1 g, 46.2 mmol) was dissolved in acetonitrile (92 mL), and imidazole (7.55 g, 111 mmol) and tert-butylchlorodimethylsilane (7.66 g, 50.8 mmol) were then added in this order, and stirred for 1 hour. At the end of the reaction, the reaction solution was quenched with methanol (5 mL) and then concentrated. The concentrate was dissolved in ethyl acetate, and the organic layer was washed with water. Subsequently, the organic layer was dried over magnesium sulfate and the solvent was distilled off under reduced pressure, and the residue was purified by silica gel column chromatography (ethyl acetate/n-hexane = 3/17) to obtain Compound 8 (15.4 g, 93.9%).
¹H-NMR (CDCl₃): δ 8.18 (d, 1H), 7.41 (t, 1H), 6.88 (dd, 1H), 5.11 (s, 2H), 4.75 (s, 2H), 3.81 (s, 3H), 0.98 (s, 9H), 0.15 (s, 6H)

Next, 2-(3-(((tert-butyldimethylsilyl)oxy)methyl)-4-nitrophenoxy)ethan-1-ol (Compound 9) was synthesized according to the following scheme:

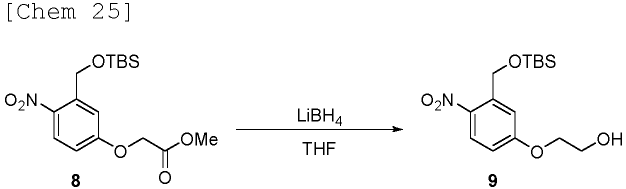

Compound 8 (15.2 g, 42.8 mmol) was dissolved in tetrahydrofuran (86 mL), and a solution of 4 mol/L of lithium borohydride in tetrahydrofuran (10.7 mL, 42.8 mmol) was then added on ice, and stirred at room temperature for 2 hours. At the end of the reaction, the mixture was quenched with acetone (10 mL) on ice, and the reaction solution was then concentrated. The concentrate was dissolved in ethyl acetate, and the organic layer was washed with a saturated aqueous solution of ammonium chloride. Subsequently, the organic layer was dried over magnesium sulfate and the solvent was distilled off under reduced pressure, and the residue was purified by silica gel column chromatography (ethyl acetate/n-hexane = 1/4 - 1/1) to obtain Compound 9 (13.1 g, 93.6%).
¹H-NMR (CDCl₃): δ 8.18 (d, 1H), 7.46 (t, 1H), 6.88 (dd, 1H), 5.12 (s, 2H), 4.19 (t, 2H), 4.03 (td, 2H), 2.04-1.98 (m, 1H), 0.98 (s, 9H), 0.16 (s, 6H)

Next, 2-(3-formyl-4-nitrophenoxy)ethyl methanesulfonate (Compound 10) was synthesized according to the following scheme:

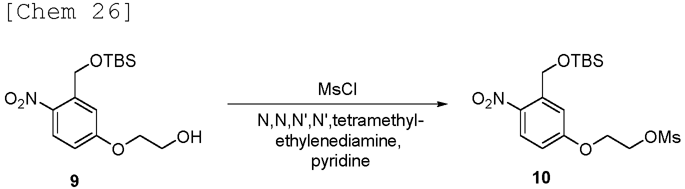

Compound 9 (3.00 g, 9.16 mmol) was dissolved in pyridine (18 mL), and N,N,N',N'-tetramethylethylenediamine (0.34 mL, 2.29 mmol) and methanesulfonyl chloride (0.851 mL, 11.0 mmol) were then added in this order under on ice, and stirred for 30 minutes. At the end of the reaction, the mixture was quenched with a saturated aqueous solution of sodium hydrogen carbonate, and then extracted with ethyl acetate. The organic layer was dried over magnesium sulfate and the solvent was distilled off under reduced pressure, and then azeotroped twice with toluene. The residue was purified by silica gel column chromatography (ethyl acetate/hexane = 1/4 → 1/1) to obtain Compound 10 (3.48 g, 93.7%).
¹H-NMR (CDCl₃): δ 8.19 (d, 1H), 7.44 (t, 1H), 6.87 (dd, 1H), 5.11 (s, 2H), 4.62-4.60 (m, 2H), 4.35-4.33 (m, 2H), 3.10 (s, 3H), 0.98 (s, 9H), 0.15 (s, 6H)

Next, Compound 11 was synthesized according to the following scheme:

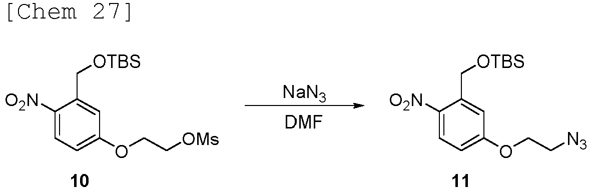

Compound 10 (3.35 g, 8.26 mmol) was dissolved in N,N-dimethylformamide (17 mL), and sodium azide (806 mg, 12.4 mmol) was added, and stirred at 80°C for 3 hours. At the end of the reaction, the mixture was cooled to room temperature, water (30 mL) was added and extracted with ethyl acetate. The organic layer was washed with water, and the organic layer was dried over magnesium sulfate and the solvent was distilled off under reduced pressure. The residue was purified by silica gel column chromatography (ethyl acetate/hexane = 1/19 → 1/9) to obtain Compound 11 (2.82 g, 96.9%).
¹H-NMR (CDCl₃): δ 8.19 (d, 1H), 7.46 (t, 1H), 6.88 (dd, 1H), 5.12 (s, 2H), 4.24 (t, 2H), 3.66 (t, 2H), 0.98 (s, 9H), 0.16 (s, 6H)

Next, (5-(2-azidoethoxy)-2-nitrophenyl)methanol (Compound 12) was synthesized according to the following scheme:

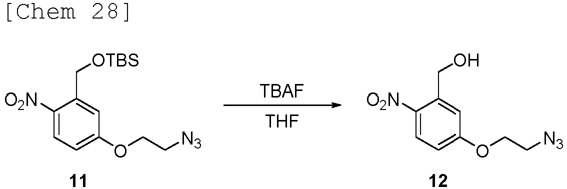

Compound 11 (1.51 g, 4.28 mmol) was dissolved in tetrahydrofuran (21.4 mL), and a solution of 1.0 mol/L of tetrabutylammonium fluoride in tetrahydrofuran (8.56 mL, 8.56 mmol) was added and stirred at room temperature for 1 hour. The reaction solution was diluted with ethyl acetate, and then washed with water, and the organic layer was dried over anhydrous sodium sulfate and concentrated. The concentrate was purified by medium pressure silica gel column chromatography (Biotage^{®} Sfar Silica HC D, 25 g, 10-25 w/w% AcOEt in Hexane) to obtain Compound 12 (0.68 g, 2.85 mmol, 66.7%).
¹H-NMR (CDCl₃): δ 8.19 (d, 1H), 7.27 (d, 1H), 6.93 (dd, 1H), 5.01 (d, 2H), 4.26 (t, 2H), 3.67 (t, 2H), 2.54 (t, 1H)

### <Synthesis of Nucleoside Derivative [O⁶-(2-nitro-5-methoxybenzyl)guanosine (Compound 15)] Having Introduced 2-nitro-5-methoxybenzyl alcohol (3)>

First, O⁶-(2-nitro-5-methoxybenzyl)-2',3',5'-tri-O-tert-butyldimethylsilylguanosine (Compound 14) was synthesized according to the following scheme:

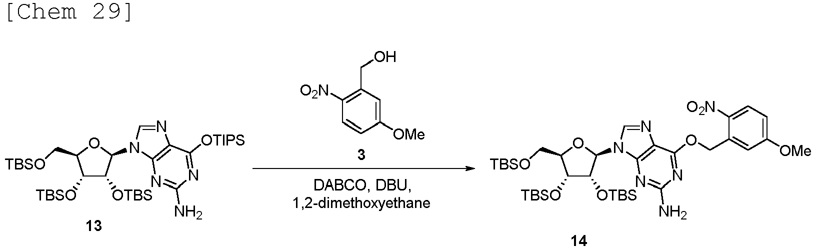

Compound 13 (6.05 g, 6.78 mmol) and Compound 3 (1.86 g, 10.2 mmol) were dissolved in 1,2-dimethoxyethane (67.8 mL), and molecular sieves 4A (3.00 g) were added, and stirred at room temperature for 1 hour. 1,4-Diazabicyclo[2.2.2]octane (1.52 g, 13.6 mmol) was added, and stirred at room temperature for 1 hour, and 1,8-diazabicyclo[5.4.0]undec-7-ene (2.03 mL, 13.6 mmol) was then added and stirred at room temperature for 15 hours. After removing the molecular sieves by filtration, the filtrate was diluted with ethyl acetate and washed with 1 mol/L of hydrochloric acid and saturated sodium hydrogen carbonate. The organic layer was dried over anhydrous sodium sulfate and concentrated. The concentrate was then purified by medium pressure silica gel column chromatography (Biotage^{®} Sfar Silica HC D, 25 g, 10-20 w/w% AcOEt in Hexane) to obtain Compound 14 (4.20 g, 5.31 mmol, 78.3%).
¹H-NMR (CDCl₃): δ 8.19 (d, 1H), 8.01 (s, 1H), 7.30 (d, 1H), 6.87 (dd, 1H), 6.02 (d, 1H), 5.97 (d, 1H), 5.91 (d, 1H), 4.88 (s, 2H), 4.53 (t, 1H), 4.30 (t, 1H), 4.10 (dd, 1H), 3.99 (dd, 1H), 3.84 (s, 3H), 3.78 (dd, 1H), 0.95 (s, 9H), 0.93 (s, 9H), 0.14 (s, 3H), 0.13 (s, 3H), 0.11 (s, 3H), 0.10 (s, 3H), -0.02 (s, 1H), -0.16 (s, 1H)

Then, O⁶-(2-nitro-5-methoxybenzyl)guanosine (Compound 15) was synthesized according to the following scheme:

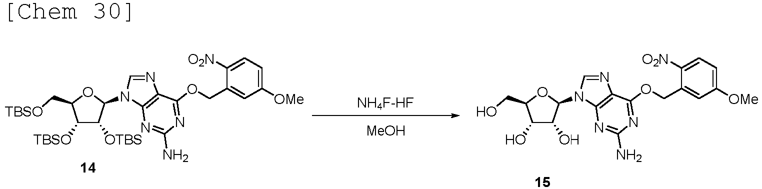

Compound 14 (4.20 g, 5.31 mmol) was dissolved in methanol (53.1 mL), and acidic ammonium fluoride (3.03 g, 53.1 mmol) was added and heated with stirring at 50°C for 11 hours. After cooling, the precipitated solid was collected by filtration and washed with methanol (3.26 g). The resulting solid was crystallized from deionized water to obtain Compound 15 (2.28 g, 5.08 mmol, 95.8%).
¹H-NMR (CDCl₃): δ 8.19 (d, 1H), 8.01 (s, 1H), 7.30 (d, 1H), 6.87 (dd, 1H), 6.02 (d, 1H), 5.97 (d, 1H), 5.91 (d, 1H), 4.88 (s, 2H), 4.53 (t, 1H), 4.30 (t, 1H), 4.10 (dd, 1H), 3.99 (dd, 1H), 3.84 (s, 3H), 3.78 (dd, 1H), 0.95 (s, 9H), 0.93 (s, 9H), 0.14 (s, 3H), 0.13 (s, 3H), 0.11 (s, 3H), 0.10 (s, 3H), -0.02 (s, 1H), -0.16 (s, 1H)

### <Synthesis of Nucleoside Derivative [O⁶-(2-nitro-5-propargyloxybenzyl)guanosine (Compound 17)] Having Introduced 2-nitro-5-propargyloxybenzyl alcohol (4)>

First, O⁶-(2-nitro-5-propargyloxybenzyl)-2',3',5'-tri-O-tert-butyldimethylsilylguanosine (Compound 16) was synthesized according to the following scheme:

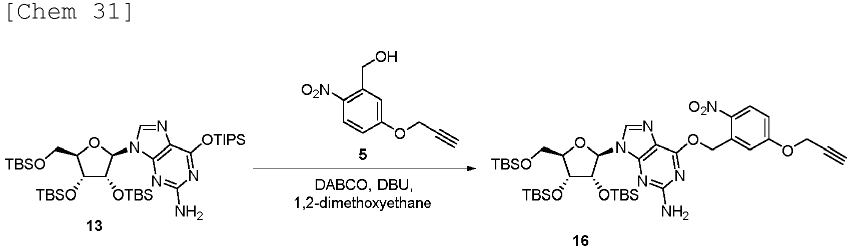

Compound 13 (2.5 g, 2.8 mmol) and Compound 5 (0.70 g, 3.4 mmol) were dissolved in 1,2-dimethoxyethane (28 mL), and molecular sieves 4A (5.0 g) were added, and stirred at room temperature for 1 hour. 1,4-Diazabicyclo[2.2.2]octane (0.63 mg, 5.6 mmol) was added, and stirred at room temperature for 1 hour. Then, 1,8-diazabicyclo[5.4.0]undec-7-ene (0.84 mL, 5.6 mmol) was added, and stirred at room temperature for 12 hours. After removing the molecular sieves by filtration, the filtrate was diluted with ethyl acetate and washed with water. The organic layer was dried over anhydrous sodium sulfate and concentrated. The concentrate was then purified by medium pressure silica gel column chromatography (Biotage^{®} Sfar Silica HC D, 25 g, 20 w/w% AcOEt in Hexane) to obtain Compound 16 (1.60 g, 1.96 mmol, 70%).
¹H-NMR (CDCl₃): δ 8.20 (d, 1H), 8.02 (s, 1H), 7.35 (d, 1H), 6.97 (dd, 1H), 6.03 (d, 1H), 5.97 (d, 1H), 5.91 (d, 1H), 4.94 (s, 2H), 4.71 (d, 1H), 4.52 (t, 1H), 4.29 (t, 1H), 4.10 (dd, 1H), 3.98 (dd, 1H), 3.78 (dd, 1H), 2.46 (t, 1H), 0.96 (s, 9H), 0.92 (s, 9H), 0.81 (s, 9H), 0.14 (s, 3H), 0.13 (s, 3H), 0.11 (s, 3H), 0.10 (s, 3H), -0.02 (s, 3H), - 0.15 (s, 3H)

Next, O⁶-(2-nitro-5-propargyloxybenzyl)guanosine (Compound 17) was synthesized according to the following scheme:

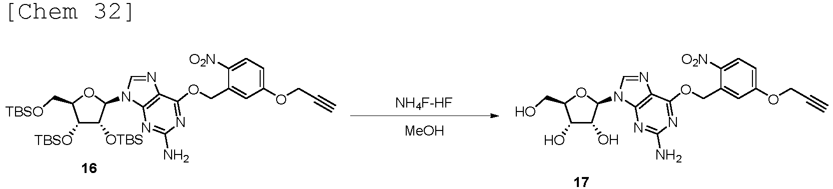

Compound 16 (1.58 g, 1.94 mmol) was dissolved in methanol (19.4 mL), and acidic ammonium fluoride (1.11 g, 19.4 mmol) was added and heated at 50°C for 12 hours with stirring. After cooling, the precipitated solid was collected by filtration and washed with methanol (1.07 g). The resulting solid was crystallized from deionized water to obtain Compound 17 (0.90 g, 1.9 mmol, 98%).
¹H-NMR (DMSO-d₆): δ 8.27 (d, 1H), 8.16 (s, 1H), 7.27 (d, 1H), 7.21 (dd, 1H), 6.52 (s, 2H), 5.92 (d, 1H), 5.88 (d, 1H), 5.81 (d, 1H), 5.43 (d, 1H), 5.14 (d, 1H), 5.09 (t, 1H), 4.96 (d, 2H), 4.50 (dd, 1H), 4.12 (dd, 1H), 3.90 (dd, 1H), 3.64 (dt, 1H), 3.60 (t, 1H), 3.54 (dt, 1H)

### <Synthesis of Nucleoside Derivative [O⁶-(2-nitro-5-(2-azidoethoxy)benzyl)guanosine (Compound 19)] Having Introduced (5-(2-azidoethoxy)-2-nitrophenyl)methanol (10)>

First, O⁶-(2-nitro-5-(2-azidoethoxy)benzyl)-2',3',5'-tri-O-tert-butyldimethylsilylguanosine (Compound 18) was synthesized according to the following scheme:

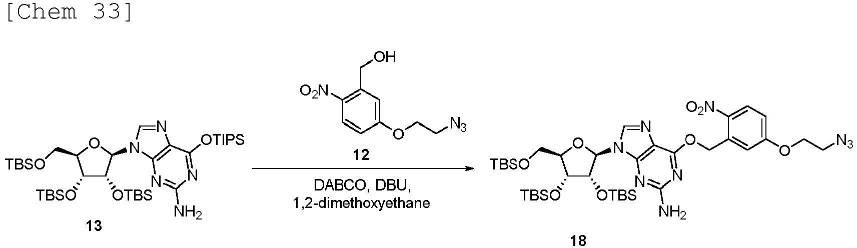

Compound 13 (2.56 g, 2.87 mmol) and Compound 12 (873 mg, 5.74 mmol) were dissolved in 1,2-dimethoxyethane (39.2 mL), and molecular sieves 4A (5.12 g) were added and stirred at room temperature for 1 hour. 1,4-Diazabicyclo[2.2.2]octane (644 mg, 5.74 mmol) was added, and stirred at room temperature for 1 hour, and 1,8-diazabicyclo[5.4.0]undec-7-ene (0.858 mL, 5.74 mmol) was then added, and stirred at room temperature for 12 hours. After removing the molecular sieves by filtration, the filtrate was diluted with ethyl acetate and washed with water. The organic layer was dried over anhydrous sodium sulfate, concentrated, and then purified by medium pressure silica gel column chromatography (Biotage^{®} Sfar Silica HC D, 25 g, 20 w/w% AcOEt in Hexane) to obtain Compound 18 (1.74 g, 2.06 mmol, 71.7%).
¹H-NMR (CDCl₃): δ 8.19 (d, 1H), 8.01 (s, 1H), 7.33 (d, 1H), 6.91 (dd, 1H), 6.04 (d, 1H), 5.96 (d, 1H), 5.92 (d, 1H), 5.03 (s, 2H), 4.52 (t, 1H), 4.29 (t, 1H), 4.18 (t, 2H), 4.11 (dd, 1H), 3.98 (dd, 1H), 3.78(dd, 1H), 3.61 (t, 2H), 0.96 (s, 9H), 0.92 (s, 9H), 0.80 (s, 9H), 0.14 (s, 3H), 0.13 (s, 3H), 0.11 (s, 3H), 0.10 (s, 3H), -0.03 (s, 3H), - 0.17 (s, 3H)

Next, O⁶-(2-nitro-5-(2-azidoethoxy)benzyl)guanosine (Compound 19) was synthesized according to the following scheme:

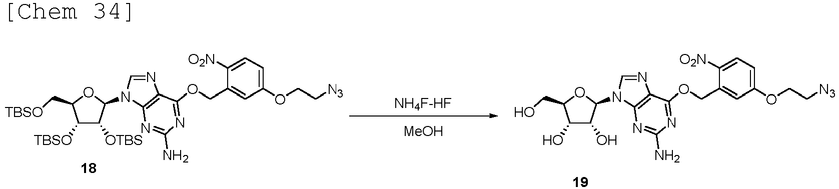

Compound 18 (1.70 g, 2.01 mmol) was dissolved in methanol (20.1 mL), and acidic ammonium fluoride (1.15 g, 20.1 mmol) was added and heated at 50°C for 12 hours with stirring. After cooling, the precipitated solid was collected by filtration and washed with methanol (1.07 g). The resulting solid was crystallized from deionized water to obtain Compound 19 (0.91 g, 1.8 mmol, 90%).
¹H-NMR (DMSO-d₆): δ 8.26 (d, 1H), 8.16 (s, 1H), 7.26 (d, 1H), 7.19 (dd, 1H), 6.53 (s, 2H), 5.92 (d, 1H), 5.88 (d, 1H), 5.81 (d, 1H), 5.42 (d, 1H), 5.14 (d, 1H), 5.09 (t, 1H), 4.49 (dd, 1H), 4.30 (t, 2H), 4.12 (dd, 1H), 3.90 (dd, 1H), 3.71 (t, 2H), 3.64 (dt, 1H), 3.55 (ddd, 1H)

### <Cleavage Test of Nucleoside Derivatives Having Introduced linkers>

A linker cleavage test was conducted by irradiating Compound 15 with ultraviolet light having a wavelength of 365 nm. Specifically, a UV lamp was placed in an aqueous solution of Compound 15, and irradiated with ultraviolet light having a wavelength of 365 nm at room temperature for 2 hours to investigate whether the following photocleavage reaction would proceed:

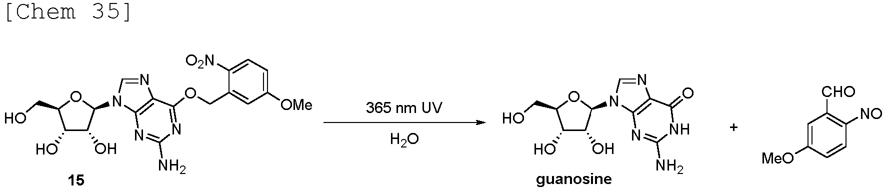

The photocleavage reaction as described above was evaluated by analyzing an aqueous solution of Compound 15 by HPLC before and after irradiation with ultraviolet light, and the results are shown in FIG. 3.

As shown in FIG. 3, after irradiation with ultraviolet light, the peak of Compound 15 disappeared and a new peak appeared. A spike test was conducted on the components of this peak, and analysis based on the data obtained from the PDA detector revealed that it was guanosine. This was able to confirm that the photocleavage reaction as described above was proceeding.

Although no cleavage test was conducted on Compounds 17 and 19, it is presumed that the photocleavage reaction will proceed in a similar manner because they have the same photocleavage structure as Compound 15.

### (2. Model Experiment on Binding of Linker to Insoluble Support and Cleavage of Linker)

To conduct model experiments on the binding of linkers to insoluble supports and the cleavage of linkers, oligonucleotide derivatives with specific linkers introduced were synthesized and bound to insoluble supports, and cleavage tests were then conducted.

As oligonucleotide derivatives, a dimer (Compound 30) and a 21-mer (Compound 31) having the following sequences were synthesized:
Sequence of dimer (Compound 30): 5'-QT-3';
Sequence of 21-mer (Compound 31): 5'-QTT TTT TTT TTT TTT TTT TTT-3'.

Q represents guanosine having an introduced linker that is cleaved by light (Compound 26).

### <Synthesis of Linker-Introduced Guanosine Amidite [O⁶-(2-nitro-5-propargyloxybenzyl)-N²-phenoxyacetyl-5'-O-dimethoxytrityl-2'-O-methylguanosine-3'-(2-cyanoethyl)-N,N-diisopropylphosphoramidite (Compound 26)]>

According to the following scheme, O⁶-(2-nitro-5-propargyloxybenzyl)-N²-phenoxyacetyl-5'-O-dimethoxytrityl-2'-O-methylguanosine 3'-amidite (Compound 26) was synthesized:

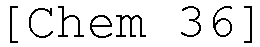

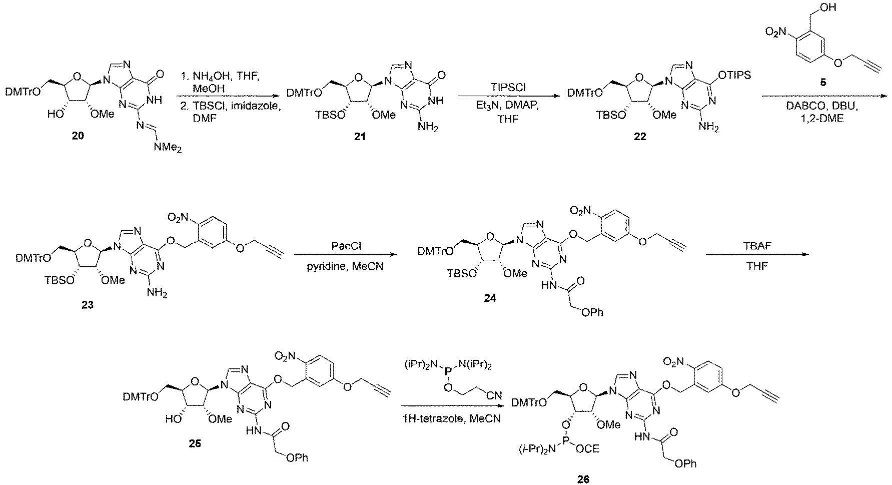

Compound 20 (Bioorganic & Medicinal Chemistry (2013), 21 (22), 6886-6892) (6.14 g, 9.38 mmol) was dissolved in methanol (93.8 mL) and tetrahydrofuran (30 mL), and 28 w/w% of aqueous ammonia (50 mL) was added and stirred at room temperature for 16 hours. Then, 28 w/w% of aqueous ammonia (50 mL) was further added and stirred at room temperature for 25 hours, and the reaction solution was concentrated. The concentrate was azeotroped three times with N,N-dimethylformamide, and the residue was dissolved in N,N-dimethylformamide (18.8 mL). Tert-butylchlorodimethylsilane (2.12 g, 14.1 mmol) and imidazole (1.92 g, 28.1 mmol) were added and stirred at room temperature for 21 hours. The reaction solution was diluted with ethyl acetate and washed with water, and the precipitated solid was then collected by filtration and washed. The organic layer of the filtrate was concentrated, and the precipitated solid was collected by filtration and washed. The obtained solids were combined and dried in vacuum to obtain Compound 21 (5.98 g, 8.38 mmol, 89.3%).

Compound 21 (5.93 g, 8.31 mmol) was then dissolved in dichloromethane (55.4 mL), and 2,4,6-triisopropylbenzenesulfonyl chloride (4.03 g, 13.3 mmol), triethylamine (1.85 mL, 13.3 mmol), and 4-dimethylaminopyridine (101 mg, 0.831 mmol) were added, and stirred at room temperature for 48 hours. The reaction solution was diluted with chloroform and washed with a saturated aqueous solution of sodium hydrogen carbonate. The organic layer was dried over anhydrous sodium sulfate and concentrated, and the concentrate was then purified by medium pressure silica gel column chromatography (Biotage^{®} Sfar Silica HC D, 25 g, 20 w/w% AcOEt in Hexane) to obtain Compound 22 (7.52 g, 7.67 mmol, 92.4%).

Compound 22 (6.00 g, 6.12 mmol) and Compound 5 (1.52 g, 7.34 mmol) were dissolved in 1,2-dimethoxyethane (61 mL), and molecular sieves 4A (12.0 g) were added and stirred at room temperature for 1 hour. 1,4-Diazabicyclo[2.2.2]octane (1.37 mg, 12.2 mmol) was added, and stirred at room temperature for 1 hour, and 1,8-diazabicyclo[5.4.0]undec-7-ene (1.83 mL, 12.2 mmol) was then added, and stirred at room temperature for 15 hours. After removing the molecular sieves by filtration, the filtrate was diluted with ethyl acetate and washed with water. The organic layer was dried over anhydrous sodium sulfate and concentrated, and the concentrate was then purified by medium pressure silica gel column chromatography (Biotage^{®} Sfar Silica HC D, 50 g, 20-35 w/w% AcOEt in Hexane) to obtain Compound 23 (4.09 g, 4.53 mmol, 74.0%).

Compound 23 (2.0 g, 2.2 mmol) was then dissolved in acetonitrile (11 mL) and pyridine (4.4 mL), and phenoxyacetyl chloride (0.37 mL, 2.7 mmol) was added on ice, and stirred for 1 hour under the same conditions. The reaction solution was returned to room temperature, and further stirred for 24 hours, and deionized water (0.5 mL) was then added and concentrated. Ethyl acetate was added to the concentrate, and washed with a saturated aqueous solution of sodium hydrogen carbonate, and the organic layer was dried over anhydrous sodium sulfate and concentrated. The concentrate was purified by medium pressure silica gel column chromatography (Biotage^{®} Sfar Silica HC D, 25 g, 25-50 w/w% AcOEt in Hexane) to obtain Compound 24 (1.84 g, 1.77 mmol, 80.0%).

Compound 24 (1.80 g, 1.74 mmol) was then dissolved in tetrahydrofuran (8.68 mL), and a solution of 1.0 mol/L tetrabutylammonium fluoride tetrahydrofuran (2.60 mL, 2.60 mmol) was added and stirred at room temperature for 30 minutes. After concentrating the reaction solution, ethyl acetate was added to the concentrate, washed with saturated saline, and the organic layer was dried over anhydrous sodium sulfate and concentrated. The concentrate was purified by medium pressure silica gel column chromatography (Biotage^{®} Sfar Silica HC D, 25 g, 1 w/w% MeOH in CHCl₃; fractions containing by-products were repurified under the same conditions) to obtain Compound 25 (1.31 g, 1.42 mmol, 81.8%).

Compound 25 (1.03 g, 1.12 mmol) was then dissolved in acetonitrile (5.58 mL), and 2-cyanoethyl N,N,N',N'-tetraisopropylphosphorodiamidite (0.53 mL, 1.67 mmol) and 1H-tetrazole (117 mg, 1.67 mmol) were added, and stirred at room temperature for 3 hours. The reaction solution was diluted with ethyl acetate, washed with water, and the organic layer was then dried over anhydrous sodium sulfate and concentrated. The concentrate was purified by medium pressure silica gel column chromatography (Yamazen Silica Gel 2L, 3.0 × 20.0 cm, 55 g, 25-33-50 w/w% AcOEt in Hexane). After concentrating the fraction containing the target product, the concentrate was dissolved in dichloromethane (3 mL) and added dropwise to hexane (500 mL). The resulting precipitate was filtered, washed (hexane), and then dried in vacuum to obtain Compound 26 (0.89 g, 0.79 mmol, 71.0%).

HRMS (ESI) m/z: theoretical value of 1121.4179 as a [M-H]⁻ ion of C₅₉H₆₂N₈O₁₃P⁻; measured value of 1121.4127.

### <Synthesis of Insoluble Support Having Azido group (Compound 29) >

An insoluble support having an azido group (Compound 29) was synthesized according to the following scheme:

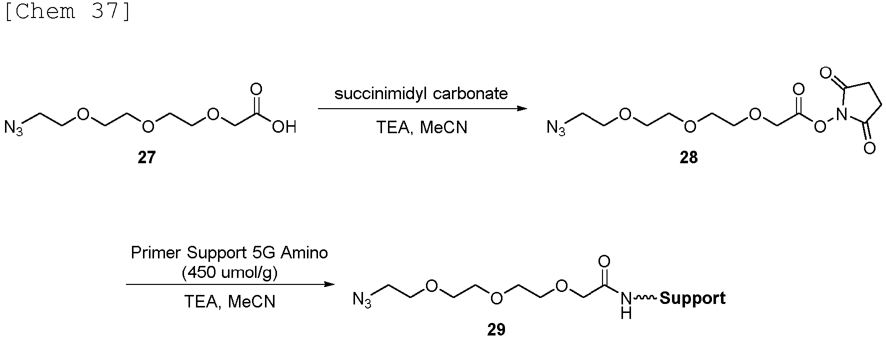

Compound 27 (100 mg, 0.429 mmol) and triethylamine (0.0717 mL, 0.515 mmol) were dissolved in acetonitrile (1.95 mL), and di(N-succinimidyl) carbonate (132 mg, 0.515 mmol) was added, and stirred at room temperature for 2 hours. The reaction solution was diluted with ethyl acetate and washed with water, and the organic layer was then dried over anhydrous sodium sulfate and concentrated. The resulting crude Compound 28 was azeotroped three times with acetonitrile, and the residue was dissolved in acetonitrile (10 mL), and triethylamine (0.0717 mL, 0.515 mmol) and Cytiva Primer support^{™} 5G amino (1.23 g, 0.45 mmol/g) were added, and stirred at room temperature for 17 hours. After confirming that the raw material was not present in the supernatant by TLC, the insoluble substances were collected by filtration. It was then washed with acetonitrile and dried in vacuum (1.29 g). To the resulting support were added Cap A [1-methylimidazole-acetonitrile (2:8)] (5 mL), Cap B1 [acetic anhydride-acetonitrile (4:6)] (2.5 mL), and Cap B2 [2,6-lutidine-acetonitrile (4:6)] (2.5 mL) were added and stirred at room temperature for 18 hours. The insoluble substance was collected by filtration, and then washed with acetonitrile and dried in vacuum to obtain an insoluble support (Compound 29) (1.28 g, 0.335 mmol/g assuming that all azide linkers were bound).

### <Synthesis of Oligonucleotide Derivatives>

Using the amidite of the compound (Compound 26) synthesized above, dT amidite, and a solid phase support, two types of oligonucleotide derivatives (dimer (Compound 30) and 21-mer (Compound 31)) were synthesized using an automatic nucleic acid synthesizer by the phosphoramidite method. At this time, the amidite of Compound 26 and dT amidite were diluted with acetonitrile to 0.15 mol/L. Other synthesis conditions were as follows:
Support: Primer support^{™} 5G T350;
Instrument: Akta oligopilot 10;
Synthesis scale: 50 µmol;
Amidite: Q amidite 23 (0.15 mol/L of acetonitrile solution, 3 equivalents), dT (0.15 mol/L of acetonitrile solution, 1.8 equivalents); and
Recycle time: 12 minutes.

The solid phase support obtained by the above synthesis was then transferred to a glass sealed tube, and 28 w/w% ammonia water (10 mL) was added, and heated at 60°C for 18 hours with stirring. After cooling, the solid phase support was removed by filtration and washed with 50 w/w% ethanol (100 mL). The filtrate and the washed solution were combined and concentrated, and then diluted in measuring cylinder with deionized water to 50 mL total.

### <Binding of Oligonucleotide Derivatives (Compounds 30 and 31) to Insoluble Support (Compound 29) (Click Reaction)>

According to the following scheme, a click reaction was carried out between a dimeric oligonucleotide derivative (Compound 30) and an insoluble support (Compound 29).

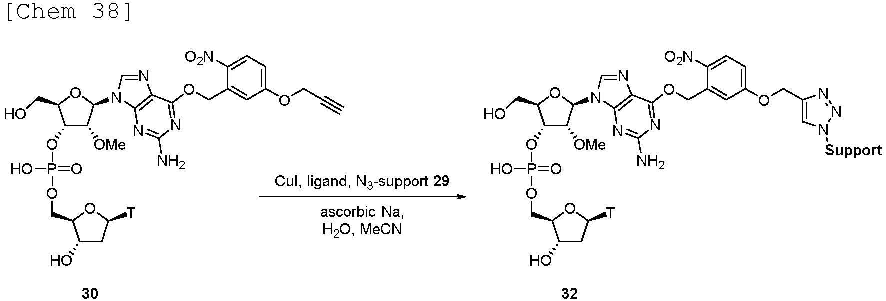

A dimeric oligonucleotide derivative (Compound 30) (3.37 µmol), copper (I) iodide (12.8 mg in 50 w/w% MeCN, 3.37 mL), tris[(1-benzyl-1H-1,2,3-triazol-4-yl)methyl]amine (TBTA) (71.5 mg), and sodium ascorbate (66.8 mg) were mixed together, and an insoluble support (Compound 29) (100 mg, 33.5 µmol) was then added and stirred at room temperature for 2 hours to carry out a click reaction. The insoluble support (reactant) was filtered off, washed with MeCN, and then dried in vacuum (104 mg). The resulting insoluble support was suspended in acetonitrile (1 mL) and an aqueous 100 mmol/L disodium ethylenediaminetetraacetate solution (3 mL) and stirred at room temperature for 16 hours. The insoluble support was filtered off, washed with deionized water and then washed with acetonitrile, and dried in vacuum to obtain a conjugate of the oligonucleotide derivative and the insoluble support (Compound 32).

The solution (supernatant) after the above click reaction was analyzed by HPLC and compared with the HPLC results of the solution before the click reaction (aqueous solution of dimeric oligonucleotide derivative). The results are shown in FIG. 4.

As shown in FIG. 4, the peak of the dimeric oligonucleotide derivative disappeared in the solution after the click reaction, indicating that the reaction of the dimeric oligonucleotide derivative (Compound 30) with the insoluble support (Compound 29) progressed.

Similarly, the click reaction was carried out between a 21-mer oligonucleotide derivative (Compound 31) and an insoluble support (Compound 29) according to the following scheme:

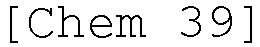

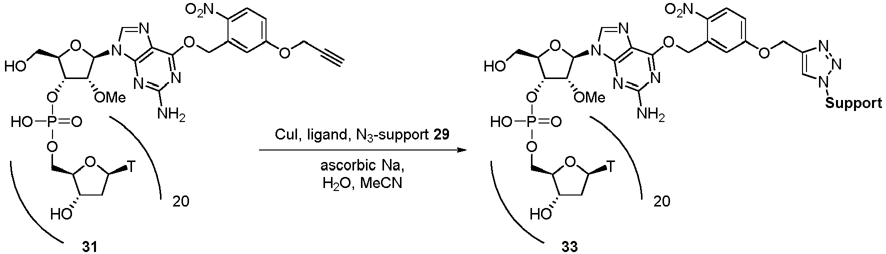

A 21-mer oligonucleotide derivative (Compound 31) (1.12 mL), deionized water (2.24 mL), copper (I) iodide (12.8 mg in 50 w/w% MeCN, 3.37 mL), tris [(1-benzyl-1H-1,2,3-triazol-4-yl)methyl]amine (TBTA) (71.5 mg), and sodium ascorbate (66.8 mg) were mixed, and an insoluble support (29) (100 mg, 33.5 µmol) was then added and stirred at room temperature for 6 hours to carry out a click reaction. Subsequently, the insoluble support (reactant) was filtered off, washed with MeCN, and then dried in vacuum. The resulting insoluble support was suspended in acetonitrile (1 mL) and an aqueous 100 mmol/L disodium ethylenediaminetetraacetate solution (3 mL) and stirred at room temperature for 16 hours. The insoluble support was filtered off, washed with deionized water and then washed with acetonitrile, and dried in vacuum to obtain a conjugate of the oligonucleotide derivative and the insoluble support (Compound 33).

### <Cleavage Test of Conjugates of Oligonucleotide Derivatives and Insoluble Supports (Compounds 32 and 33)>

The conjugate of the oligonucleotide derivative and the insoluble support (Compound 32) were suspended in acetonitrile (3 mL) and deionized water (7 mL), irradiated with ultraviolet light having a wavelength of 365 nm (room temperature, 3 hours), and the supernatant was analyzed by HPLC over time to investigate whether the following photocleavage reaction progressed. The HPLC results are shown in FIG. 5.

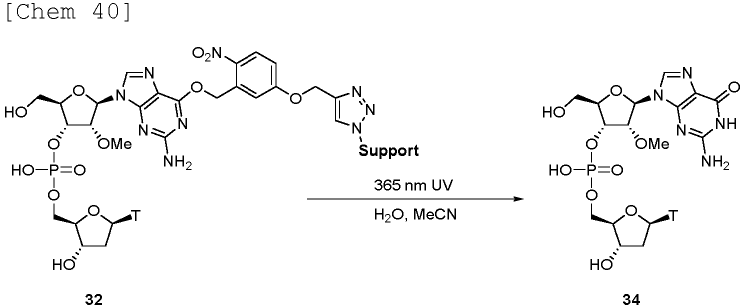

As shown in FIG. 5, a new peak was observed in HPLC after irradiation with ultraviolet light, and analysis by LC/MS confirmed that this was Compound 34.

Similarly, the conjugate of the oligonucleotide derivative and the insoluble support (Compound 33) was suspended in acetonitrile (3 mL) and deionized water (7 mL), irradiated with ultraviolet light having a wavelength of 365 nm (room temperature, 3 hours), and the supernatant was analyzed by HPLC over time to investigate whether the following photocleavage reaction progressed. The HPLC results are shown in FIG. 6.

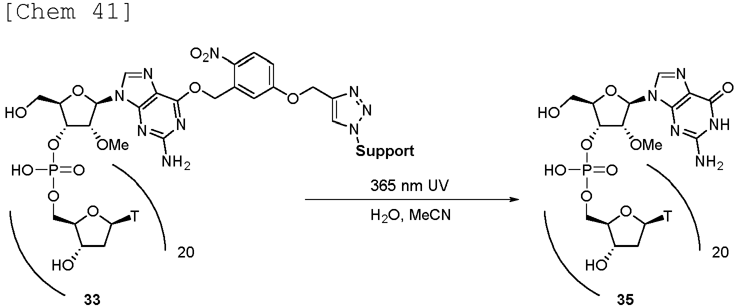

As shown in FIG. 6, a new peak was observed in HPLC after irradiation with ultraviolet light, and analysis by LC/MS confirmed that this was Compound 35.

### (3. Experiments Using mRNA Cap Analogues)

The model experiments 1 and 2 described above showed that after the oligonucleotide derivatives were bound to the insoluble supports, the linkers could be cleaved by irradiation with ultraviolet light. Therefore, several mRNA cap analogues were actually synthesized and various experiments were conducted.

### <Synthesis 1 of mRNA Cap Analog (Compound 47)>

First, Compound 36 was synthesized according to the following scheme:

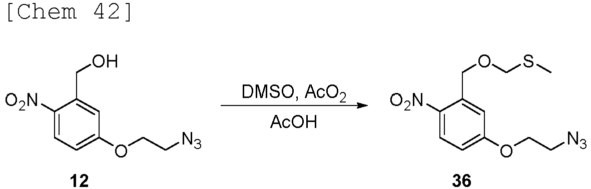

Compound 12 (10.7 g, 44.9 mmol) was dissolved in acetic acid (103 mL), and dimethyl sulfoxide (64 mL) and acetic anhydride (85 mL) were then added in this order on ice, and stirred at room temperature for 42 hours. At the end of the reaction, the reaction solution was dropped into an ice-cooled aqueous 10 mmol/L sodium hydroxide solution (400 mL). At end of the dropping, the mixture was stirred at room temperature for 1.5 hours. After extracting it with ethyl acetate, the organic layer was dried over magnesium sulfate and the solvent was distilled off under reduced pressure. The residue was purified by silica gel column chromatography (ethyl acetate/hexane = 1/19 → 1/4) to obtain Compound 36 (9.58 g, 71.5%).
¹H-NMR (CDCl₃): δ 8.19 (d, 1H), 7.32 (t, 1H), 6.91 (dd, 1H), 5.03 (s, 2H), 4.82 (s, 2H), 4.25 (t, 2H), 3.66 (t, 2H), 2.21 (s, 3H)

Compound 38 was then synthesized according to the following scheme:

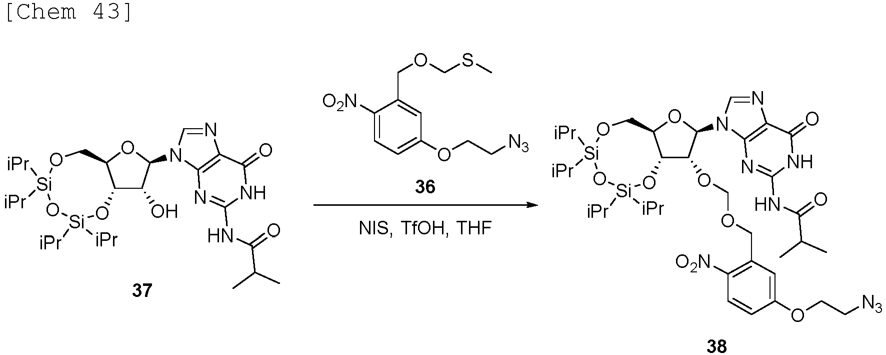

Compound 37 (2.50 g, 4.20 mmol) and Compound 36 (1.88 g, 6.29 mmol) were dissolved in tetrahydrofuran (16.8 mL), and molecular sieves 3A (2.50 g) were added, and cooled to -40°C. Trifluoromethanesulfonic acid (557 µL, 6.29 mmol) and N-iodosuccinimide (1.42 g, 6.29 mmol) were added in this order, and stirred at the same temperature for 7 hours. A saturated aqueous solution of sodium hydrogen carbonate and a saturated aqueous solution of sodium thiosulfate were added and stirred, and then extracted with ethyl acetate. The organic layer was dried over anhydrous magnesium sulfate and concentrated. The concentrate was purified by silica gel column chromatography (1-2 w/w% methanol-chloroform) and then dried in vacuum to obtain Compound 38 (2.89 g, 3.42 mmol, 81.4%).
¹H-NMR (DMSO-d₆): δ 11.97 (s, 1H), 11.49 (s, 1H), 8.09 (d, 1H), 8.04 (s, 1H), 7.01 (dd, 1H), 6.96 (d, 1H), 5.83 (d, 1H), 5.14 (d, 1H), 5.04 (d, 1H), 5.02 (d, 1H), 4.95 (d, 1H), 4.60 (dd, 1H), 4.49 (dd, 1H), 4.19 (t, 2H), 4.09 (dd, 1H), 4.01 (dt, 1H), 3.94 (dd, 1H), 3.68 (t, 2H), 2.67 (m, 1H), 1.13 (d, 1H), 1.10 (d, 1H), 1.06-0.86 (m, 28H)

Compound 39 was synthesized according to the following scheme:

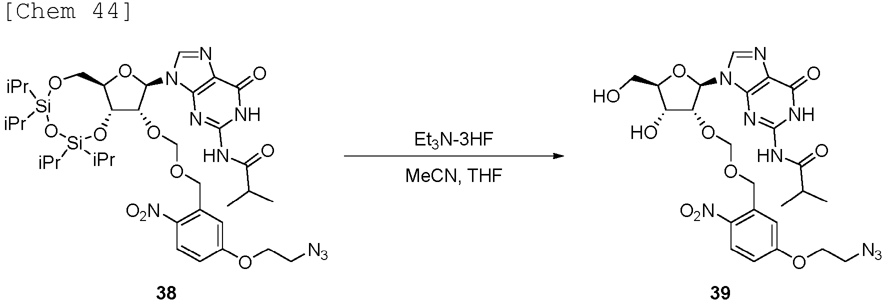

Compound 38 (2.89 g, 3.42 mmol) was dissolved in acetonitrile (11.4 mL) and tetrahydrofuran (11.4 mL), and triethylamine trihydrofluoride (557 µL, 3.42 mmol) was added and stirred at room temperature for 20 hours. The reaction solution was diluted with chloroform and washed with a saturated aqueous solution of sodium hydrogen carbonate. After the mixture was extracted with chloroform from an aqueous layer, the organic layer was dried over anhydrous magnesium sulfate and concentrated. The concentrate was purified by silica gel column chromatography (2-5 w/w% methanol-chloroform) and then dried in vacuum to obtain Compound 39 (1.87 g, 3.10 mmol, 90.7%).
¹H-NMR (DMSO-d₆): δ 11.77 (s, 1H), 11.50 (s, 1H), 8.26 (s, 1H), 8.04 (d, 1H), 6.99 (dd, 1H), 6.70 (d, 1H), 5.94 (d, 1H), 5.38 (d, 1H), 5.14 (t, 1H), 4.91 (d, 1H), 4.88 (d, 1H), 4.77 (d, 1H), 4.69 (d, 1H), 4.68 (t, 1H), 4.30 (m, 1H), 4.23 (m, 2H), 3.96 (m, 1H), 3.73 (m, 2H), 3.60 (m, 2H), 2.68 (m, 1H), 1.14 (d, 3H), 1.09 (d, 3H)

Compound 41 was then synthesized according to the following scheme:

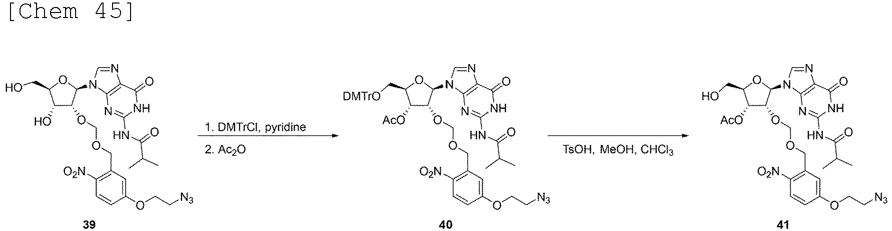

Compound 39 (1.87 g, 2.87 mmol) was azeotroped three times with pyridine, and the residue was dissolved in pyridine (9.6 mL). 4,4'-Dimethoxytrityl chloride (1.17 g, 3.44 mmol) was added and stirred at room temperature for 15 hours. Acetic anhydride (678 µL, 7.71 mmol) was added to this reaction solution and further stirred at room temperature for 16 hours. Methanol (5 mL) was added to the reaction solution and concentrated, and ethyl acetate was then added to the concentrate and washed with a saturated aqueous solution of sodium hydrogen carbonate. The organic layer was dried over anhydrous magnesium sulfate and concentrated, and the concentrate was azeotroped three times with toluene to obtain crude Compound 40.

The crude Compound 40 was then dissolved in chloroform (28.7 mL) and cooled to 0°C. A solution of 4-toluenesulfonic acid hydrate (998 mg) in methanol (9.57 mL) was added and stirred at the same temperature for 1 hour. A saturated aqueous solution of sodium hydrogen carbonate was added to the reaction solution and stirred, and the organic layer was then dried over anhydrous magnesium sulfate and concentrated. The concentrate was purified by silica gel column chromatography (2-5 w/w % methanol-chloroform) and then dried in vacuum to obtain Compound 41 (1.88 g, 2.71 mmol, 94.4%).
¹H-NMR (DMSO-d₆): δ 11.79 (s, 1H), 11.54 (s, 1H), 8.29 (d, 1H), 8.04 (d, 1H), 7.00 (dd, 1H), 6.68 (d, 1H), 5.90 (d, 1H), 5.42 (d, 1H), 5.37 (t, 1H), 4.91 (dd, 1H), 4.82 (d, 1H), 4.78 (d, 1H), 4.66 (d, 1H), 4.61 (d, 1H), 4.25 (m, 2H), 4.17 (m, 1H), 3.73 (m, 2H), 3.67 (m, 2H), 2.71 (m, 1H), 2.12 (s, 3H), 1.15 (d, 3H), 1.09 (d, 3H)

Compound 42 was then synthesized according to the following scheme:

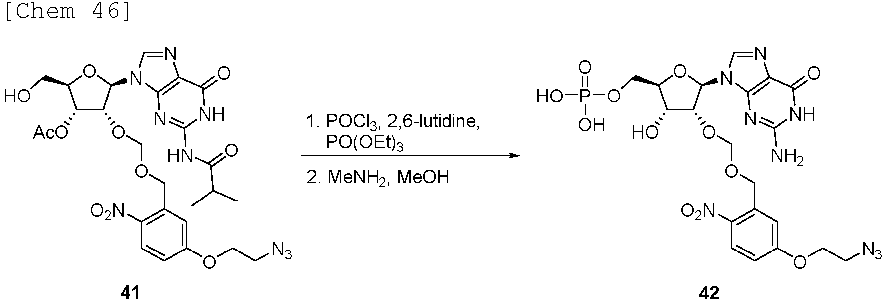

Compound 41 (3.95 g, 6.12 mmol) and 2,6-lutidine (1.8 mL, 15.3 mmol) were dissolved in triethyl phosphate (19.0 mL) and cooled to -10°C. Phosphorus oxychloride (1.39 mL, 15.3 mmol) was added and stirred at the same temperature for 9 hours, and 1.0 mol/L of triethylammonium hydrogen carbonate (30 mL) was then added and stirred at the same temperature for 16 hours. Deionized water (50 mL) was added to the reaction solution, and then extracted five times with chloroform. The organic layers were combined and concentrated, and dissolved in 100 mmol/L of triethylammonium acetate (50 mL). The product was purified by ODS silica gel column chromatography (100 mmol/L of triethylammonium acetate to 50 w/w% of acetonitrile, 100 mmol/L of triethylammonium acetate). The fraction containing the target product was concentrated to a small volume, and then extracted three times with chloroform. The organic layers were combined and concentrated, and the concentrate was dissolved in methanol (30.6 mL). To this solution was added a solution of 40 w/w% methylamine in methanol (10.2 mL) and stirred at room temperature for 24 hours. After concentrating the reaction solution, the concentrate was brought into a solution of 200 mmol/L triethylammonium acetate (500 mL) and purified by ODS silica gel column chromatography (YAMAZEN ODS-SM 30 µm, L, 3.0 × 16.5 cm, 37 g, 100 mmol/L of triethylammonium acetate to 50 w/w% of acetonitrile, 100 mmol/L of triethylammonium acetate). After concentrating the fraction containing the target product, the concentrate was azeotroped 10 times with deionized water. The resulting crystalline residue was dissolved in ethanol (75 mL) with heating, and the precipitated crystals were collected by filtration and dried to obtain Compound 42 (2.41 g, 3.37 mmol, 55.1%). The crystal mother liquor was re-purified by ODS silica gel column chromatography (YAMAZEN ODS-SM 30 µm, L, 3.0 × 16.5 cm, 37 g, 100 mmol/L of triethylammonium acetate to 50 w/w% of acetonitrile, 100 mmol/L of triethylammonium acetate), thereby further obtaining 0.28 g of Compound 42.
¹H-NMR (DMSO-d₆): δ 10.51 (s, 1H), 8.10 (s, 1H), 7.94 (s, 1H), 7.00 (dd, 1H), 6.94 (d, 1H), 6.46 (s, 2H), 5.85 (d, 1H), 4.88 (d, 1H), 4.82 (d, 1H), 4.77 (d, 1H), 4.72 (m, 1H), 4.68 (d, 1H), 4.34 (m, 1H), 4.29 (m, 2H), 4.03 (m, 1H), 3.89 (m, 2H), 3.73 (m, 2H), 2.94 (q, 6H), 1.13 (t, 9H)

Compound 43 was then synthesized according to the following scheme:

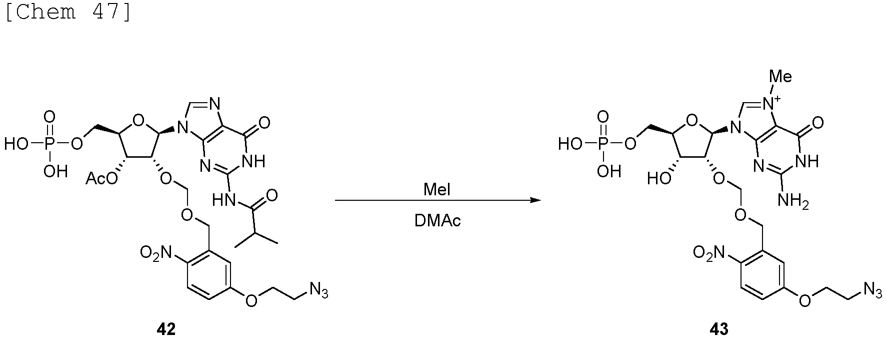

Compound 42 (2.20 g, 3.08 mmol) was dissolved in N,N-dimethylacetamide (30.8 mL), and iodomethane (959 µL) was added, and stirred at room temperature for 24 hours. The reaction solution was concentrated, and deionized water was added to the concentrate, and the precipitated solid was then collected by filtration, washed with water, and then dried in vacuum to obtain Compound 43 (1.56 g, 2.49 mmol, 80.8%).
¹H-NMR (DMSO-d₆): δ 9.86 (s, 1H), 8.13 (d, 1H), 7.52 (s, 2H), 7.05-7.01 (m, 2H), 5.98 (d, 1H), 4.99 (s, 2H), 4.86 (s, 2H), 4.69 (t, 1H), 4.41 (t, 1H), 4.29 (m, 2H), 4.16 (s, 1H), 4.05 (m, 1H), 3.94 (m, 1H), 3.90 (s, 3H), 3.72 (m, 2H)

Compound 45 was then synthesized according to the following scheme:

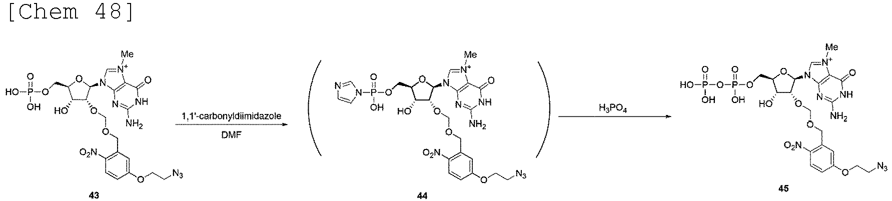

Compound 43 (400 mg, 0.637 mmol) was dissolved in N,N-dimethylformamide (3.19 mL), and tributylamine (0.163 mL, 0.637 mmol) and 1,1'-carbonyldiimidazole (114 mg, 0.701 mmol) were added, and stirred at room temperature for 5 hours. Deionized water (5 µL) was added, and then stirred for 30 minutes, and 2-tributylammonium phosphate (2.55 mmol) dissolved in N,N-dimethylformamide was added, and stirred at room temperature for 19 hours. Deionized water (10 mL) was added, and stirred at room temperature for 5 hours, and then purified by ODS silica gel column chromatography (YAMAZEN ODS-SM 30 µm, L, 3.0 × 16.5 cm, 37 g, deionized water to 50 w/w% acetonitrile). The fraction containing the target product was concentrated, and the concentrate was azeotroped three times with acetonitrile and dried in vacuum. Compound 45 (308 mg, 0.381 mmol, 59.7%) was obtained.
HRMS (ESI) m/z: theoretical value of 706.1029 as a [M-H]⁻ion of C₂₁H₂₆N₉O₁₅P₂⁻; measured value of 706.0864.

Compound 47 was then synthesized according to the following scheme:

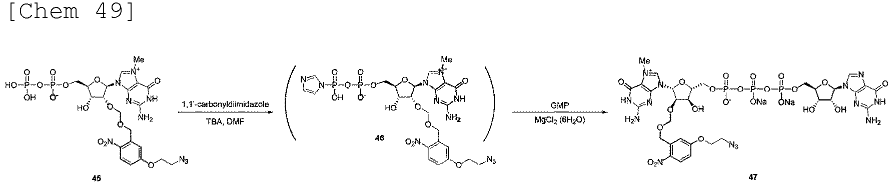

Compound 45 (121 mg, 0.150 mmol) was dissolved in N,N-dimethylformamide (0.75 mL), and tributylamine (0.163 mL, 0.637 mmol) and 1,1'-carbonyldiimidazole (36.5 mg, 0.225 mmol) were added, and stirred at room temperature for 5 hours. After adding deionized water (10 µL), the mixture was stirred for 30 minutes, and a solution of guanosine 5'-monophosphate monotributylamine salt (123 mg, 0.225 mmol) dissolved in N,N-dimethylformamide (0.50 mL) and a solution of magnesium chloride hexahydrate dissolved in N,N-dimethylformamide (0.50 mL) were then added in this order, and stirred at room temperature for 17 hours. The reaction solution was diluted with deionized water (10 mL) and then purified by ODS silica gel column chromatography (YAMAZEN ODS-SM 30 µm, L, 3.0 × 16.5 cm, 37 g, deionized water to 50 w/w% acetonitrile). The fraction containing the target product was concentrated, and the concentrate was azeotroped three times with acetonitrile and dried in vacuum to obtain Compound 47 (98 mg, 0.089 mmol, 60%). HRMS (ESI) m/z: theoretical value of 1051.1503 as a [M-H]⁻ion of C₃₁H₃₈N₁₄O₂₂P₃⁻; measured value of 1051.1542.

### <Synthesis 2 of mRNA Cap Analog (Compound 47)>

Compound 47 was synthesized using a method different from that described above.

First, Compound 50 was synthesized according to the following scheme:

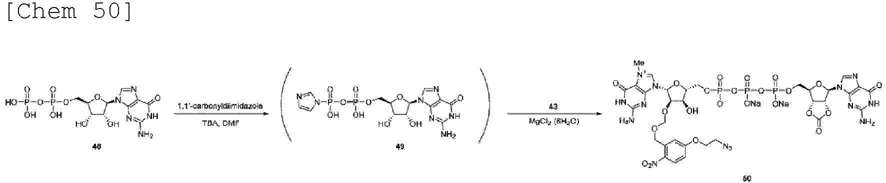

Tributylamine (163 µL, 637 µmol) was added to an aqueous solution of Compound 48 (84.8 mmol/L, 4.51 mL, 382 µmol) and concentrated, and the concentrate was azeotroped three times with N,N-dimethylformamide. The residue was dissolved in N,N-dimethylformamide (1.59 mL), and 1,1'-carbonyldiimidazole (129 mg, 0.797 mmol) was added, and stirred at room temperature for 3 hours. Deionized water (5 µL) was added and then stirred for 30 minutes, and solutions of Compound 43 (200 mg, 319 µmol) and magnesium chloride hexahydrate (77.8 mg, 382 µmol) dissolved in N,N-dimethylformamide (0.50 mL) was added in this order, and stirred at room temperature for 22 hours. Deionized water (5 mL) was added, and the precipitated solid was collected by filtration and washed with water. Furthermore, the solid precipitated in the filtrate was collected by filtration and washed with water. The resulting solids were combined and dried in vacuum to obtain Compound 50 (313 mg, 290 µmol, 91.0%).
HRMS (ESI) m/z: theoretical value of 1077.1296 as a [M-H]⁻ion of C₃₂H₃₆N₁₄O₂₃P₃⁻; measured value of 1077.1289.

Compound 47 was then synthesized according to the following scheme:

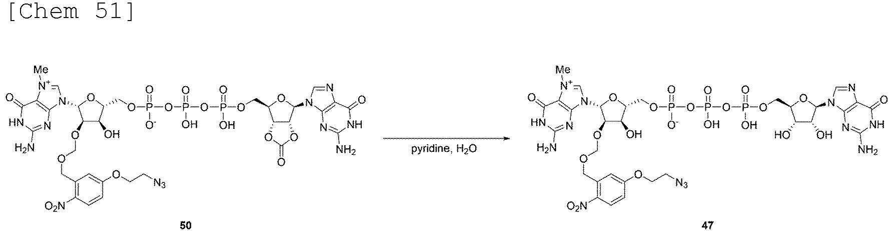

Compound 50 (200 mg, 185 µmol) was dissolved in pyridine (927 µL) and deionized water (927 µL), and heated at 60°C for 3 hours with stirring. Acetonitrile was added to the reaction solution, and the resulting precipitate was collected by filtration and washed with acetonitrile. The resulting precipitate was dissolved in deionized water (30 mL) and purified by ODS silica gel column chromatography (YAMAZEN ODS-SM, 30 µm, M, 2.3 × 12.3 cm, 14 g, 100 mmol/L of TEAA to 100 mmol/L of TEAA, 50 w/w% of acetonitrile). The fraction containing the target product was concentrated and then desalted by ODS silica gel column chromatography (YAMAZEN ODS-SM, 30 µm, M, 2.3 × 12.3 cm, 14 g, deionized water to 50 w/w% of acetonitrile). The fraction containing the target substance was concentrated to a small volume and passed through a cation exchange column (Dowex 50w × 8, Na type, 10 mL) and washed with water. The eluate and the washed solution were combined and concentrated, and the concentrate was dried in vacuum to obtain Compound 47 (101 mg, 185 µmol, 49.2%).

### <Synthesis of mRNA Cap Analog (Compound 54)>

Compound 54 was synthesized according to the following scheme:

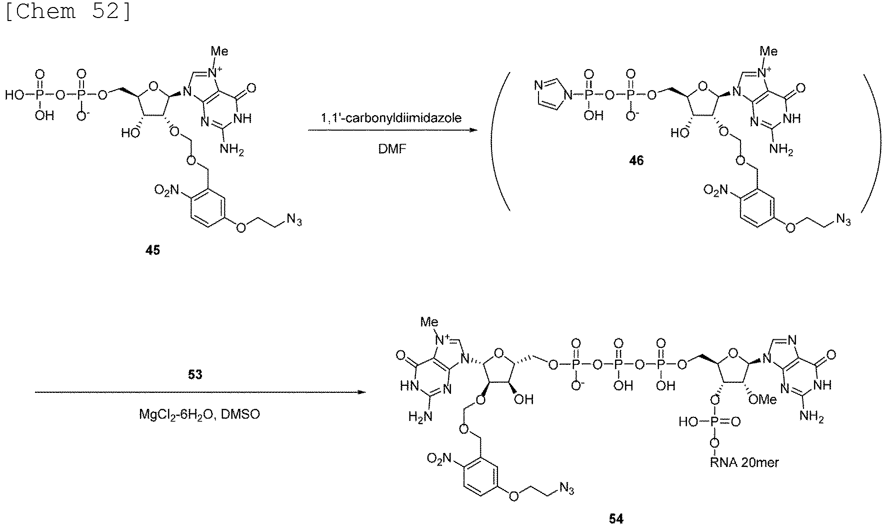

Compound 45 (20 mg, 0.025 mmol) was dissolved in N,N-dimethylformamide (0.99 mL), and 1,1'-carbonyldiimidazole (6.0 mg, 0.037 mmol) was added, and stirred at room temperature for 3 hours. After adding deionized water (10 µL), the mixture was stirred for 30 minutes, and a solution of an oligonucleotide derivative (Compound 53; 0.025 mmol; sequence 5'-G^{OMe}G^{OMe}G AGA CGC GUG UUA AAU AAC-3' in which G^{OMe} is 2'OMe-G) dissolved in dimethylsulfoxide (2 mL) and a solution of magnesium chloride hexahydrate (50 mg, 0.25 mmol) dissolved in N,N-dimethylformamide (1 mL) were then added in this order, and stirred at room temperature for 20 hours. A separately prepared solution of Compound 46 (equivalent to 0.037 mmol) in N,N-dimethylformamide (1 mL) was added and further stirred for 24 hours. The reaction solution was diluted with deionized water to 300 mL and purified by ODS silica gel column chromatography (YAMAZEN ODS-AQ, 30 µm, 2 L, 3.0 × 20.0 cm, 51 g, 100 mmol/L of TEAA to 100 mmol/L of TEAA, 50 w/w% of acetonitrile). The fraction containing the target product was concentrated and then desalted by ODS silica gel column chromatography (YAMAZEN ODS-AQ, 30 µm, 2 L, 3.0 × 20.0 cm, 51 g, deionized water to 50 w/w% of acetonitrile). The fraction containing the target product was concentrated to obtain Compound 54 as an aqueous solution (6.86 µmol, 27.4%).
HRMS (ESI) m/z: theoretical value of 7592.0590 as a [M-H]⁻ion of C₂₂₆H₂₇₈N₉₆O₁₅₉P₂₃⁻; measured value of 7592.0747.

### <Synthesis of Insoluble Support Having Cyclooctyne Group (Compound 60)>

An insoluble support having a cyclooctyne group (Compound 60) was synthesized according to the following scheme:
First, Compound 57 was synthesized according to the following scheme:

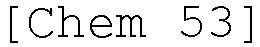

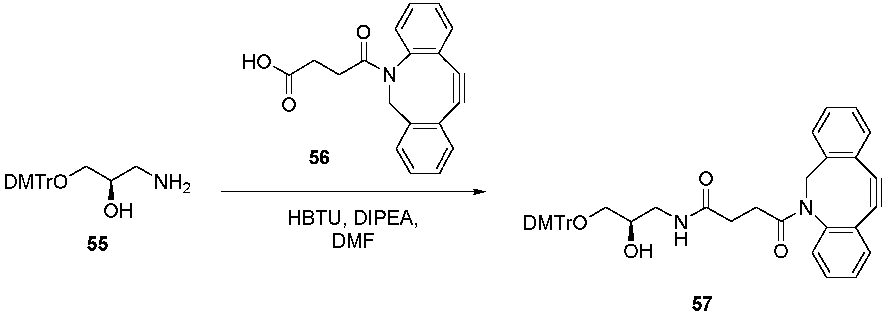

The commercially available compound 56 (388 mg, 1.27 mmol) and N,N-diisopropylethylamine (681 µmol) were dissolved in N,N-dimethylformamide (6.35 mL) and cooled on ice. 1-[bis(dimethylamino)methylene]-1H-benzotriazolium 3-oxidehexafluorophosphate (530 mg, 1.40 mmol) was added and stirred at room temperature for 1 hour. After cooling the reaction solution on ice, a solution of Compound 55 (Organic & Biomolecular Chemistry (2014), 12(14), 2263-2272) (500 mg, 1.27 mmol) in N,N-dimethylformamide (2 mL) was added and stirred at room temperature for 18 hours. The reaction solution was diluted with ethyl acetate, and then washed with a saturated aqueous solution of sodium hydrogen carbonate, and the organic layer was dried over anhydrous magnesium sulfate and concentrated. The concentrate was purified by silica gel column chromatography (50-75-100 w/w% ethyl acetate-hexane) to obtain Compound 57 (0.61 g, 0.90 mmol, 71%).
HRMS (ESI) m/z: theoretical value of 679.2814 as a [M-H]⁻ ion of C₄₃H₃₉N₂O₆⁻; measured value of 679.2819; theoretical value of 725.2868 as a [M+HCOO]⁻ ion of C₄₄H₄₁N₂O₈⁻; measured value of 725.2890; theoretical value of 739.3025 as a [M+CH₃COO]⁻ ion of C₄₅H₄₃N₂O₈⁻; measured value of 739.2993.

Compound 59 was then synthesized according to the following scheme:

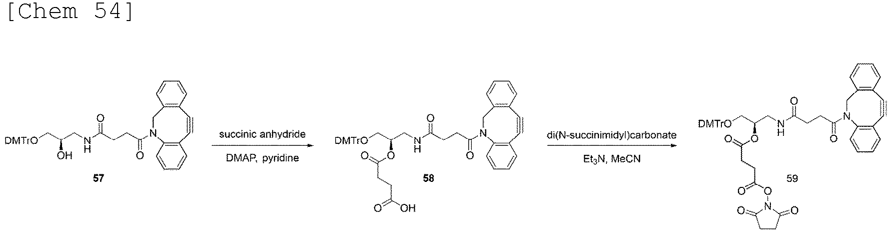

Compound 57 (0.49 g, 0.72 mmol) was dissolved in pyridine (3.6 mL), and succinic anhydride (0.14 g, 1.4 mmol) and N,N-dimethylaminopyridine (44 mg, 0.36 mmol) were added and stirred at room temperature for 18 hours. The reaction solution was concentrated, and ethyl acetate was then added to the concentrate and washed with water. The organic layer was dried over anhydrous magnesium sulfate, and the residue was dissolved in acetonitrile. Di(N-succinimidyl) carbonate (0.22 g, 0.86 mmol) and triethylamine (0.24 mL, 1.7 mmol) were added, and stirred at room temperature for 3 hours. The reaction solution was diluted with ethyl acetate, and then washed with a saturated aqueous solution of sodium hydrogen carbonate, and the organic layer was dried over anhydrous magnesium sulfate and concentrated. The concentrate was purified by silica gel column chromatography (75-100 w/w% ethyl acetate-hexane) to obtain Compound 59 (0.54 g, 0.62 mmol, 85%).
HRMS (ESI) m/z: theoretical value of 876.3138 as a [M-H]⁻ion of C₅₁H₄₆N₃O₁₁⁻; measured value of 876.3094.

Compound 60 was then synthesized according to the following scheme:

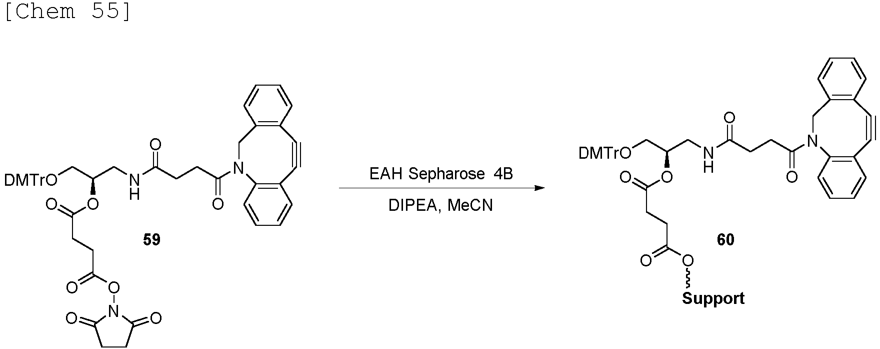

A suspension of EAH-Sepharose^{™} 4B (7-12 µmol/mL) was centrifuged at 2,000 rpm for 5 minutes (volume after centrifugation: 10 mL), and the supernatant was removed by decantation. The precipitate was suspended in acetonitrile (10 mL), and then centrifuged at 2,000 rpm for 5 minutes, and the supernatant was removed by decantation. This procedure was repeated five times. The precipitate was suspended in acetonitrile (10 mL), and N,N-diisopropylethylamine (25 µL, 0.14 mmol) and Compound 59 (61 mg, 70 µmol) were added, and stirred at room temperature for 15 hours. Furthermore, N,N-diisopropylethylamine (25 µL, 0.14 mmol) and Compound 59 (61 mg, 70 µmol) were added, and stirred at room temperature overnight. This procedure was repeated twice. To this suspension was added N-acetylimidazole (7.7 mg, 70 µmol), and stirred at room temperature for 24 hours. The suspension was centrifuged at 2,000 rpm for 5 minutes, and the supernatant was removed by decantation. The precipitate was suspended in acetonitrile (10 mL), and then centrifuged at 2,000 rpm for 5 minutes, and the supernatant was removed by decantation. This procedure was repeated five times to obtain a precipitate (Compound 60). The precipitate was made up to 10 mL with deionized water.

### <Evaluation of Amount of Cyclooctyne Unit Supported in Compound 60>

Compound 60 was treated according to the following scheme:

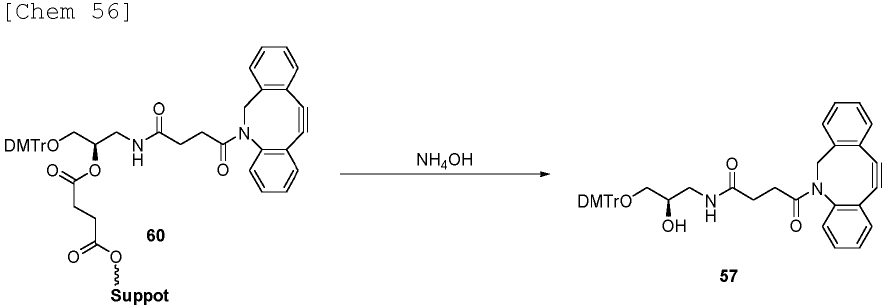

To a suspension of Compound 60 (0.5 mL) was added 28 w/w% of aqueous ammonia (1 mL) and heated at 60°C for 17 hours. After removing unnecessary materials by filtration, the filtrate and the washed solution were concentrated and made up to 10 mL with 50 w/w% of acetonitrile containing 1 w/w% of triethylamine. The Compound 57 produced was analyzed by mass spectrometry (theoretical value as AcO⁻adduct: 739.3025; measured value: 739.3027). Also, the quantification using a calibration curve indicated that the amount of the cyclooctyne unit supported on the insoluble support was 3.19 µmol/mL.

### <Binding of mRNA Cap Analog (Compound 47) to Insoluble Support having Cyclooctyne Group (Compound 60) (Click Reaction) >

According to the following scheme, a click reaction was carried out between the mRNA cap analog (Compound 47) and the insoluble support having the cyclooctyne group (Compound 60) to obtain a conjugate (Compound 61).

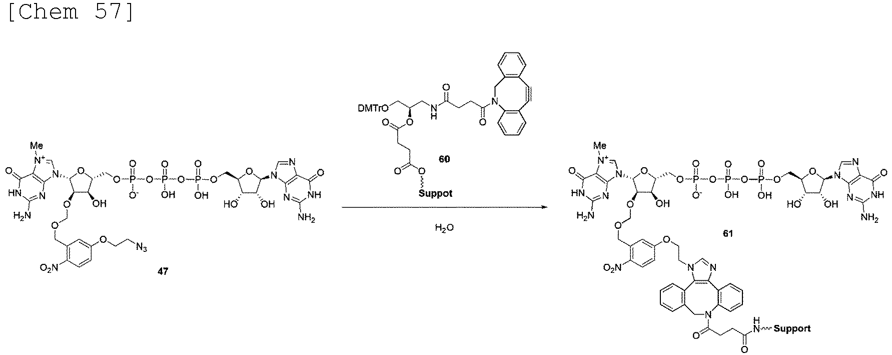

A suspension of Compound 60 (0.50 mL, 1.60 µmol) and a solution of Compound 47 (1 mmol/mL, 1.60 mL, 1.60 µmol) were mixed and stirred at room temperature for 4 days. During this time, the reaction solution was analyzed over time, and the progress of the click reaction was confirmed by HPLC by confirming that the peak of Compound 47 in the reaction solution decreased. The results are shown in FIG. 7.

### <Cleavage Test of Compound 61>

At the end of the click reaction, the suspension was centrifuged at 40,000 rpm for 5 minutes, and the supernatant was removed by decantation. The precipitate was suspended in deionized water (3 mL), and then centrifuged at 40,000 rpm for 5 minutes, and the supernatant was removed by decantation. This procedure was repeated five times. The precipitate was suspended in deionized water (5 mL), cooled to 5°C, and irradiated with ultraviolet light having a wavelength of 365 nm with stirring. Whether the following photocleavage reaction progressed was investigated by HPLC.

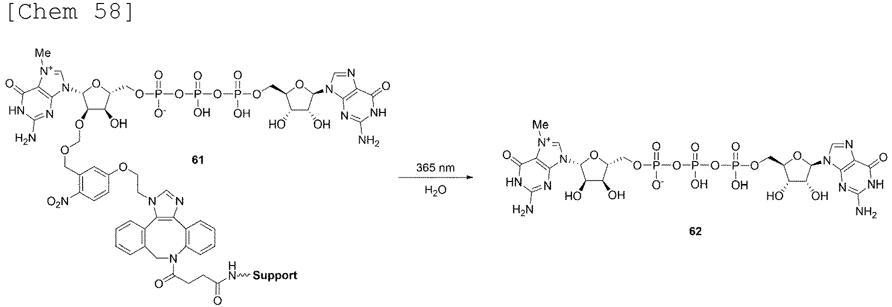

As a result, as shown in FIG. 8, it was confirmed that a new component was produced in the reaction supernatant. As a result of mass spectrometry, it was identified as Compound 62. This confirmed that the cleavage reaction by irradiation with ultraviolet light having a wavelength of 365 nm had progressed.

### <Binding of mRNA Cap Analog (Compound 54) to Insoluble Support having Cyclooctyne Group (Compound 60) (Click Reaction) >

According to the following scheme, the click reaction was carried out between the mRNA cap analog (Compound 54) and the insoluble support having the cyclooctyne group (Compound 60) to obtain a conjugate (Compound 63).

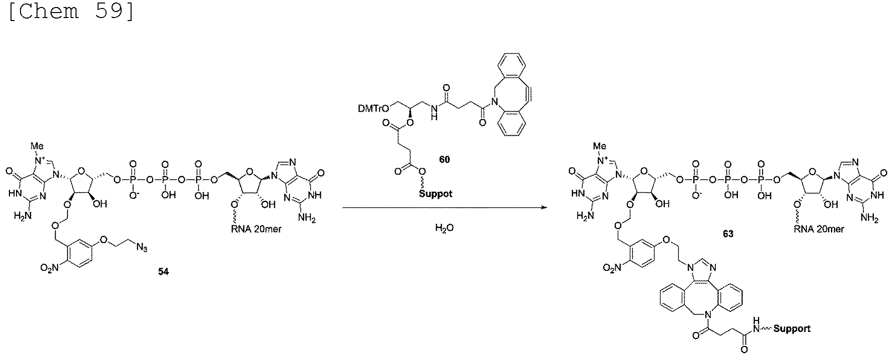

A suspension of Compound 60 (0.50 mL, 1.60 µmol), a solution of Compound 54 (0.5 mL, 0.083 µmol), and Compound 53 as an internal standard were mixed and stirred at room temperature for 4 days. During this time, the reaction solution was analyzed over time, and the progress of the click reaction was confirmed by confirming that the peak of Compound 54 decreased in the reaction solution (FIG. 9).

### <Cleavage Test of Compound 63>

At the end of the click reaction, the suspension was centrifuged at 40,000 rpm for 5 minutes, and the supernatant was removed by decantation. The precipitate was suspended in deionized water (3 mL), and then centrifuged at 40,000 rpm for 5 minutes, and the supernatant was removed by decantation. This procedure was repeated five times. The precipitate was suspended in deionized water (3 mL), cooled to 5°C, and irradiated with ultraviolet light having a wavelength of 365 nm with stirring. Whether the following photocleavage reaction progressed was investigated by HPLC.

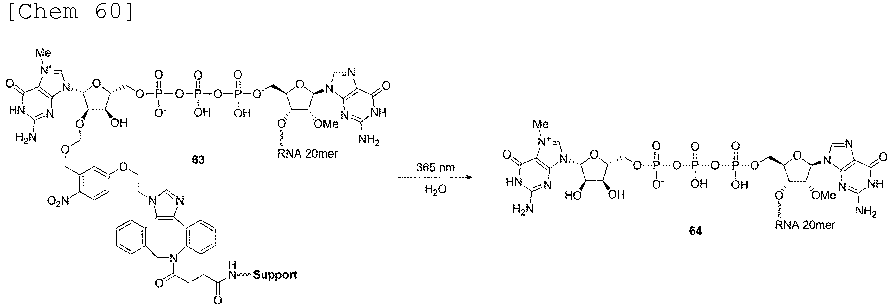

As a result, as shown in FIG. 10, a new peak was generated in HPLC after irradiation with ultraviolet light, and analysis by LC/MS confirmed that it was Compound 64. This confirmed that the cleavage reaction had progressed due to irradiation with ultraviolet light having a wavelength of 365 nm.

### <Synthesis of Insoluble Support Having Cyclooctyne Group (Compound 72)>

First, Compound 65 was synthesized according to the following scheme:

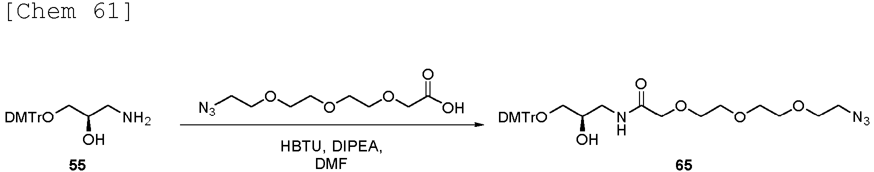

11-Azido-3,6,9-trioxaundecanoic acid (1.00 g, 4.29 mmol) and N,N-diisopropylethylamine (2.30 mL, 12.9 mmol) were dissolved in N,N-dimethylformamide (21.4 mL) and cooled on ice. 1-[Bis(dimethylamino)methylene]-1H-benzotriazolium 3-oxidehexafluorophosphate (1.79 g, 4.72 mmol) was added and stirred at room temperature for 1 hour. After cooling the reaction solution on ice, a solution of Compound 55 (1.69 g, 4.29 mmol) in N,N-dimethylformamide (10 mL) was added and stirred at room temperature for 16 hours. The reaction solution was diluted with ethyl acetate, and then washed with a saturated aqueous solution of sodium hydrogen carbonate, and the organic layer was dried over anhydrous magnesium sulfate and concentrated. The concentrate was purified by silica gel column chromatography (85 w/w% ethyl acetate-hexane to 2 w/w% methanol-ethyl acetate) to obtain Compound 65 (2.47 g, 4.06 mmol, 94.6%).
¹H-NMR (DMSO-d₆): δ 7.45-6.87 (m, 14H), 5.05 (d, 1H), 3.84 (s, 1H), 3.74 (s, 7H), 3.60-3.49 (m, 10H), 3.37 (t, 2H), 3.09 (dt, 1H), 2.93 (dd, 1H), 2.84 (dd, 1H)

Compound 67 was synthesized according to the following scheme:

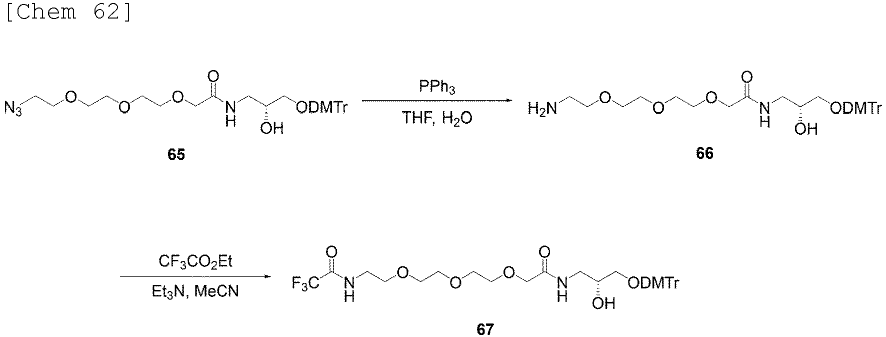

Compound 65 (0.87 g, 1.4 mmol) was dissolved in tetrahydrofuran (7.1 mL) and deionized water (0.71 mL), and triphenylphosphine (0.41 g, 1.6 mmol) was added and stirred at room temperature for 15 hours. The reaction solution was concentrated, and the concentrate was then azeotroped with acetonitrile three times. The residue was dissolved in acetonitrile (7.1 mL), and triethylamine (0.44 mL, 3.1 mmol) and ethyl trifluoroacetate (0.34 mL, 2.9 mmol) were added, and stirred at room temperature for 1 hour. The reaction solution was concentrated, and the concentrate was purified by silica gel column chromatography (75-85-100 w/w% ethyl acetate-hexane to 2 w/w% methanol-ethyl acetate) to obtain Compound 67 (0.80 g, 1.2 mmol, 82%).
¹H-NMR (DMSO-d₆) : δ 9.48 (t, 1H), 7.46-6.87 (m, 14H), 5.05 (d, 1H), 3.84 (s, 2H), 3.74 (s, 7H), 3.51-3.48 (m, 10H), 3.38-3.35 (m, 1H), 3.08 (dt, 1H), 2.93 (dd, 1H), 2.84 (dd, 1H)

Compound 69 was then synthesized according to the following scheme:

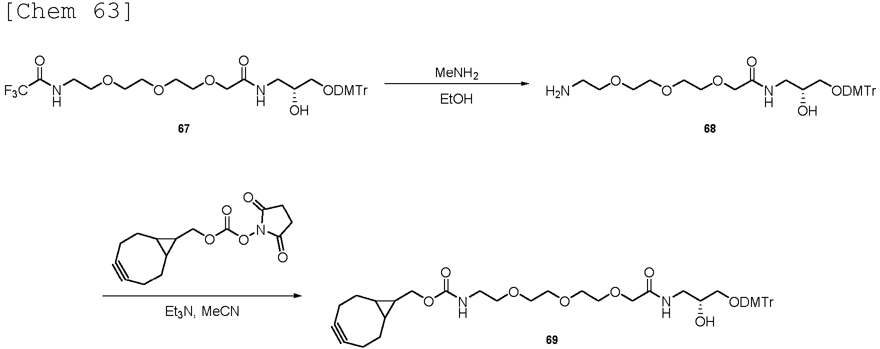

Compound 67 (0.58 g, 0.85 mmol) was placed in ethanol (3.9 mL), and a solution of 33 w/w% of methylamine in ethanol (1.6 mL) was added, and stirred at room temperature for 24 hours. The reaction solution was concentrated, and the concentrate was azeotroped three times with acetonitrile. The residue was dissolved in acetonitrile (3.9 mL), and triethylamine (0.13 mL, 0.93 mmol) and succinimidyl (1R,8S,9s)-bicyclo[6.1.0]non-4-yn-9-ylmethyl carbonate (0.23 g, 0.78 mmol) were added, and stirred at room temperature for 24 hours. The reaction solution was diluted with ethyl acetate and washed with a saturated aqueous solution of hydrogen carbonate, and the organic layer was dried over anhydrous magnesium sulfate and concentrated. The concentrate was purified by silica gel column chromatography (ethyl acetate to 1 w/w% methanol-ethyl acetate) to obtain Compound 69 (0.44 g, 0.58 mmol, 75%).
HRMS (ESI) m/z: theoretical value of 757.3706 as a [M-H]⁻ion of C₄₃H₅₃N₂O₁₀⁻; measured value of 757.3727.

Compound 71 was then synthesized according to the following scheme:

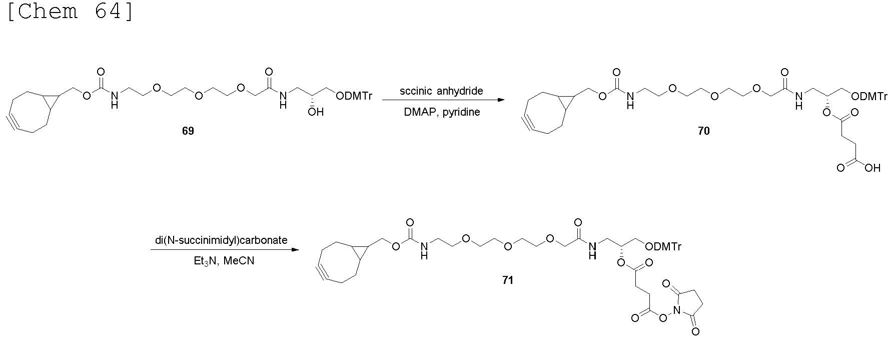

Compound 69 (0.70 g, 0.92 mmol) was dissolved in pyridine (4.6 mL), and succinic anhydride (0.18 g, 1.8 mmol) and N,N-dimethylaminopyridine (56 mg, 0.46 mmol) were added, and stirred at room temperature for 19 hours. The reaction solution was concentrated, and ethyl acetate was then added to the concentrate, and washed with an aqueous 1 mol/L citric acid solution. The organic layer was dried over anhydrous magnesium sulfate, concentrated, and then dissolved in acetonitrile (4.6 mL). Di(N-succinimidyl) carbonate (0.28 g, 1.1 mmol) and triethylamine (0.31 mL, 2.2 mmol) were added, and stirred at room temperature for 3 hours. The reaction solution was diluted with ethyl acetate, and then washed with a saturated aqueous solution of sodium hydrogen carbonate, and the organic layer was dried over anhydrous magnesium sulfate and concentrated. The concentrate was purified by silica gel column chromatography (2-5-10-30 w/w% acetone-ethyl acetate) to obtain Compound 71 (0.161 g, 0.168 mmol, 18%).
HRMS (ESI) m/z: theoretical value of 954.4030 as a [M-H]⁻ion of C₅₁H₆₀N₃O₁₅⁻; measured value 954.3997.

Compound 72 was then synthesized according to the following scheme:

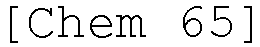

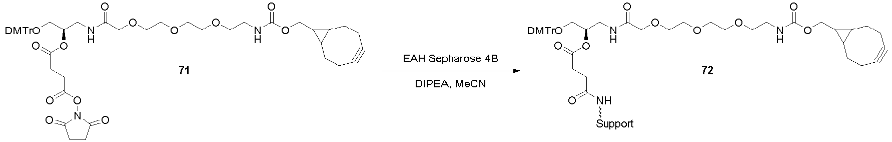

A suspension of EAH-Sepharose^{™} 4B (7-12 µmol/mL) was centrifuged at 2,000 rpm for 5 minutes (volume after centrifugation: 10 mL), and the supernatant was removed by decantation. The precipitate was suspended in acetonitrile (10 mL), and then centrifuged at 2,000 rpm for 5 minutes, and the supernatant was removed by decantation. This procedure was repeated five times. The above precipitate was suspended in acetonitrile (10 mL), and N,N-diisopropylethylamine (37 µL, 0.21 mmol) and Compound 71 (67 mg, 70 µmol) were added, and stirred at room temperature for 16 hours. Furthermore, N,N-diisopropylethylamine (37 µL, 0.21 mmol) and Compound 71 (67 mg, 70 µmol) were added, and stirred at room temperature for 24 hours. To this mixture was added N-acetylimidazole (27 mg, 0.21 mmol), and stirred at room temperature for 24 hours. The suspension was centrifuged at 4,000 rpm for 10 minutes, and the supernatant was removed by decantation. The precipitate was suspended in an aqueous 100 mmol/mL triethylammonium acetate solution (20 mL), and then centrifuged at 4,000 rpm for 10 minutes, and the supernatant was removed by decantation. This procedure was repeated three times to obtain a precipitate (Compound 72). The precipitate was adjusted to 10 mL with deionized water.

### <Evaluation of Amount of Cyclooctyne Unit supported in Compound 72>

Compound 72 was treated according to the following scheme:

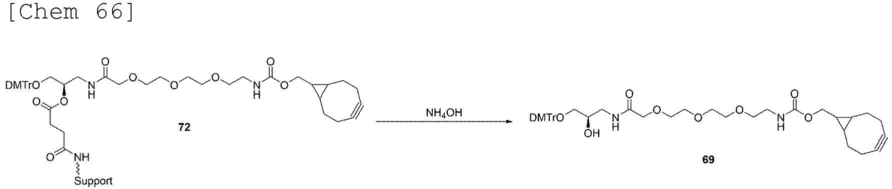

To a suspension of Compound 72 (0.5 mL) was added 28 w/w% aqueous ammonia (1 mL) and heated at 60°C for 17 hours. After removing unnecessary materials by filtration, the filtrate and the washed solution were concentrated and made up to 10 mL with 60 w/w% acetonitrile containing 1 w/w% triethylamine. The Compound 69 produced was analyzed by mass spectrometry (theoretical values: 757.3706, 803.3760 (HCO₂⁻), 817.3917 (AcO⁻); measured values: 757.3727, 803.3773 (HCO₂⁻), 817.3928 (AcO⁻)). Also, the quantification using a calibration curve indicated that the amount of the cyclooctyne unit supported on the insoluble support was 4.22 µmol/mL.

### <Binding of mRNA Cap Analog (Compound 54) to Insoluble Support Having Cyclooctyne Group (Compound 72) (Click Reaction)>

According to the following scheme, the click reaction was carried out between the mRNA cap analog (Compound 54) and the insoluble support having the cyclooctyne group (Compound 72) to obtain a conjugate (Compound 73).

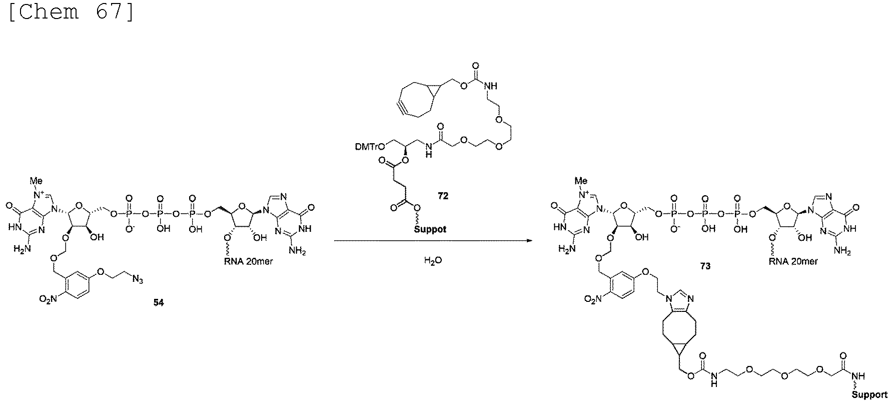

A suspension of Compound 72 (0.50 mL, 2.11 µmol), a solution of Compound 54 (0.5 mL, 0.083 µmol), and Compound 53 as an internal standard were mixed and stirred at room temperature for 4 days. During this time, the reaction solution was analyzed over time, and the progress of the click reaction was confirmed by confirming that the peak of Compound 54 deceased in the reaction solution (FIG. 11).

### <Cleavage Test of Compound 73>

The suspension after the click reaction was centrifuged at 40,000 rpm for 5 minutes, and the supernatant was removed by decantation. The precipitate was suspended in deionized water (3 mL), and then centrifuged at 40,000 rpm for 5 minutes, and the supernatant was removed by decantation. This procedure was repeated five times. The precipitate was suspended in deionized water (3 mL), cooled to 5°C, and irradiated with ultraviolet light having 365 nm with stirring, and whether the following photocleavage reaction proceeded was investigated by HPLC.

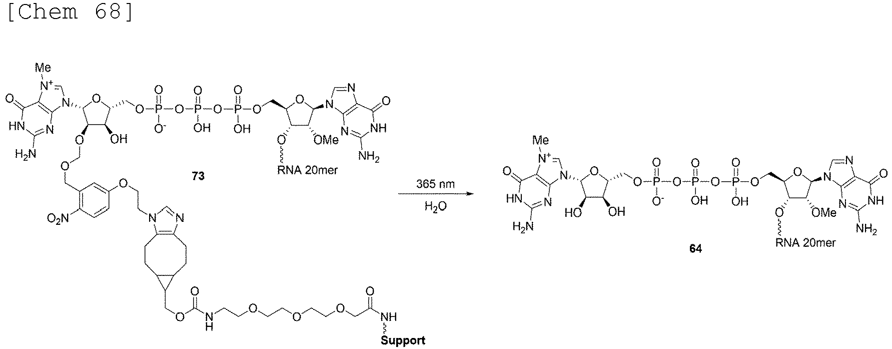

As a result, as shown in FIG. 12, a new peak was generated in HPLC after irradiation with ultraviolet light, and analysis by LC/MS confirmed that it was Compound 64. This confirmed that the cleavage reaction had progressed due to irradiation with ultraviolet light having a wavelength of 365 nm.

The above results demonstrate that it is possible to bind insoluble supports (Compounds 60 and 72) to mRNA cap analogues (Compounds 47 and 54) by the click reaction, and to cleave the linkers by irradiation with ultraviolet light.

### [Sequence Listing]

SEQ ID NO: 1: Oligonucleotide derivative (21-mer) synthesized in Examples
SEQ ID NO: 2: Oligonucleotide derivative (Compound 53) used in Examples

## Claims

1. A mRNA cap analog, comprising a linker at a sugar or a base moiety of a nucleotide, the linker comprising a functional group selected from an amino group, a carboxylic acid active ester group, an aminooxy group, a hydrazino group, a carbonyl group, a thiol group, a haloacetyl group, a Michael acceptor functional group, an alkynyl group, and a 1,3-dipole functional group, the linker having a structure cleavable by light or a fluoride ion.

2. The mRNA cap analog according to claim 1, wherein the linker has an o-nitrobenzyl ether structure or a silyl ether structure.

3. The mRNA cap analog according to claim 1 or 2, wherein the mRNA cap analog is represented by the following general formula (1) or (2): 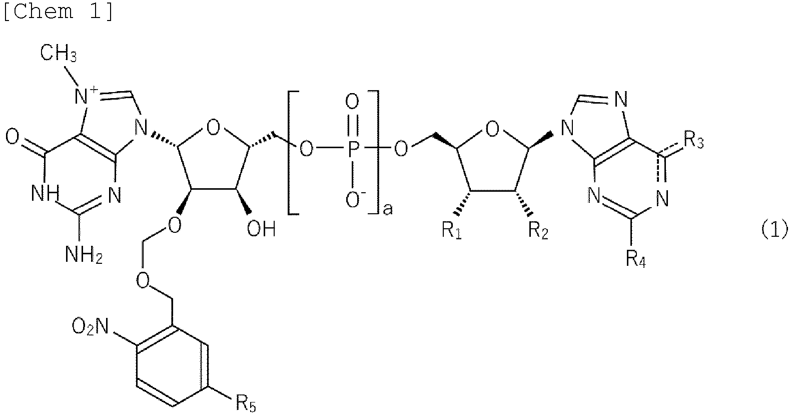 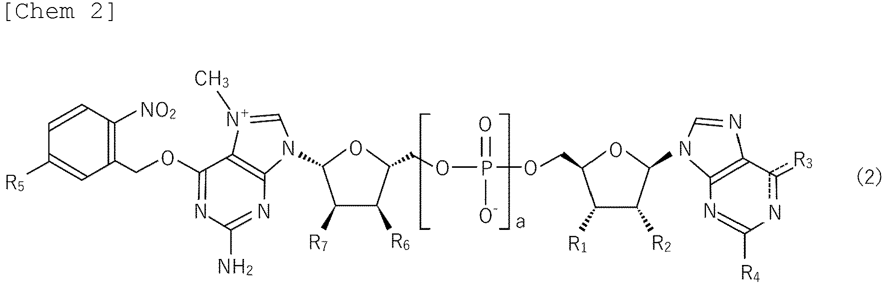 in which:
R₁ is OH, OCH₃ or a group represented by the following general formula (3): 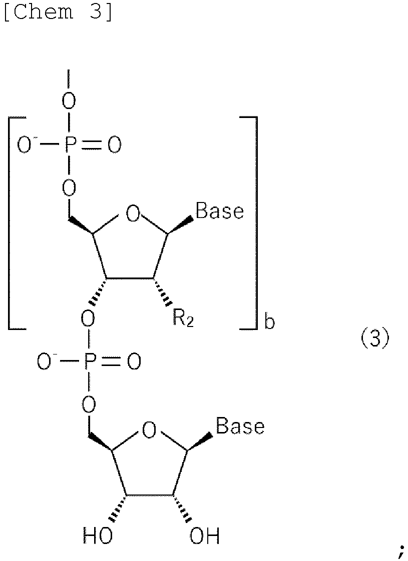
R₂ is OH or OCH₃;
R₃ is O, OH, NH₂, NHCH₃ or N(CH₃)₂, and when R₃ is O, then the bond between the C bonded to R₃ and R₃ is a double bond, and the bond between the C bonded to R₃ and the N adjacent thereto is a single bond, and when R₃ is OH, NH₂, NHCH₃ or N(CH₃)₂, then the bond between the C bonded to R₃ and R₃ is a single bond, and the bond between the C bonded to R₃ and the N adjacent thereto is a double bond;
R₄ is NH₂, NHCH₃, N(CH₃)₂ or H;
R₅ is a propargyloxy group or an azidoethoxy group;
R₆ and R₇, which may be identical or different, are OH or OCH₃;
a is 3 to 6;
b is 0 to 19; and
Base is adenine-9-yl, N6-methyladenine-9-yl, guanine-9-yl, cytosine-1-yl, 5-methylcytosine-1-yl, uracil-1-yl, uracil-5-yl or 1-methyluracil-5-yl.

4. The mRNA cap analog according to claim 3, wherein R₃ is O and R₄ is NH₂.

5. A method for producing a mRNA having a 5' cap structure, the method comprising the steps of:
mixing a mRNA cap analog comprising a linker having a functional group A and a structure cleavable by light or fluoride ion at a sugar or a base moiety of a nucleotide with a nucleoside triphosphate, a template DNA and an RNA polymerase, and obtaining a preparation comprising a mRNA having a 5' cap structure by *in vitro* transcription;
mixing the preparation with an insoluble support having a functional group B capable of reacting with the functional group A to generate a conjugate of the mRNA and the insoluble support;
filtering and washing the conjugate; and
applying light or a fluoride ion to the conjugate to cleave the linker and isolate the mRNA having the 5' cap structure.

6. The method according to claim 5, wherein the functional groups A and B are a combination of an amino group and a carboxylic acid activated ester, a combination of an aminooxy group or a hydrazino group and a carbonyl group, a combination of a thiol group and a thiol group, a haloacetyl group or a Michael acceptor functional group, or a combination of an alkynyl group and a 1,3-dipole functional group.

7. The method according to claim 5 or 6, wherein the linker has an o-nitrobenzyl ether structure or a silyl ether structure.

8. The method according to claim 5, wherein the mRNA cap analog is represented by the following general formula (1) or (2): 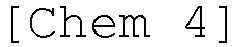 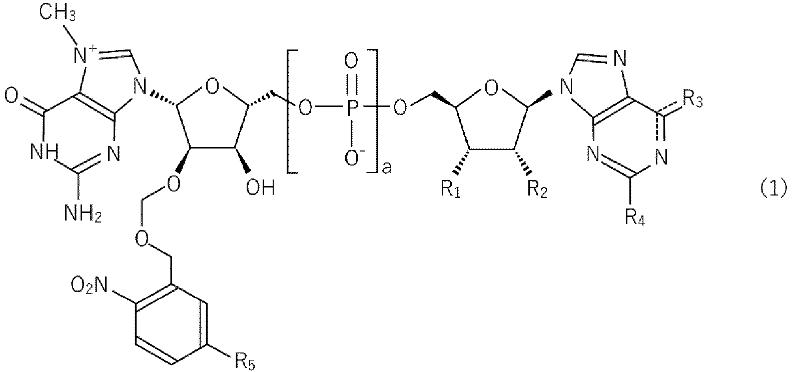 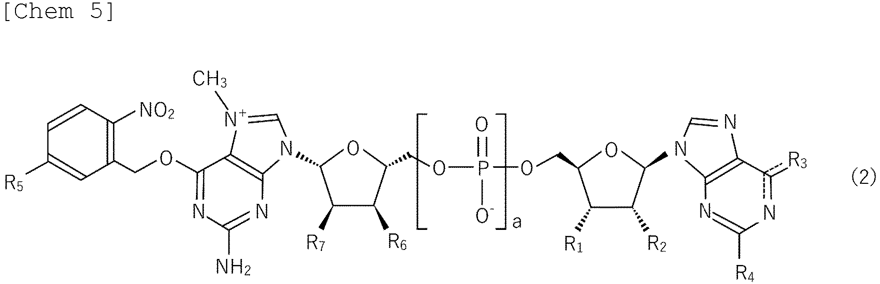 in which:
R₁ is OH, OCH₃ or a group represented by the following general formula (3): 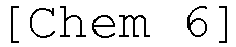 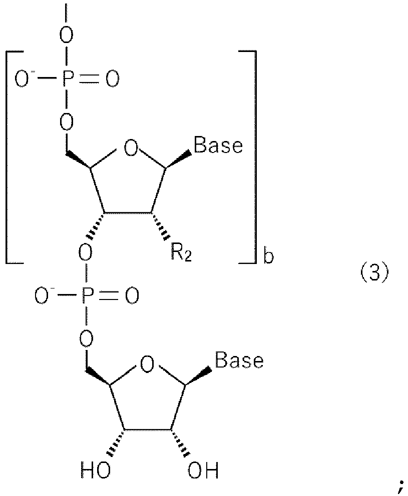
R₂ is OH or OCH₃;
R₃ is O, OH, NH₂, NHCH₃ or N(CH₃)₂, and when R₃ is O, then the bond between the C bonded to R₃ and R₃ is a double bond, and the bond between the C bonded to R₃ and the N adjacent thereto is a single bond, and when R₃ is OH, NH₂, NHCH₃ or N(CH₃)₂, then the bond between the C bonded to R₃ and R₃ is a single bond, and the bond between the C bonded to R₃ and the N adjacent thereto is a double bond;
R₄ is NH₂, NHCH₃, N(CH₃)₂ or H;
R₅ is a propargyloxy group or an azidoethoxy group;
R₆ and R₇, which may be identical or different, are OH or OCH₃;
a is 3 to 6;
b is 0 to 19; and
Base is adenine-9-yl, N6-methyladenine-9-yl, guanine-9-yl, cytosine-1-yl, 5-methylcytosine-1-yl, uracil-1-yl, uracil-5-yl or 1-methyluracil-5-yl.

9. The method according to claim 8, wherein R₃ is O and R₄ is NH₂.

10. The method according to claim 8 or 9, wherein, when R₅ is a propargyloxy group, then the insoluble support is represented by the following formula (4): 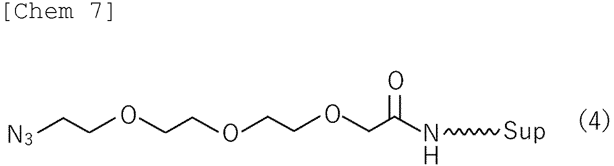 in which Sup is an insoluble support structural moiety.

11. The method according to claim 8 or 9, wherein, when R₅ is an azidoethoxy group, the insoluble support is represented by the following formula (5), (6) or (7): 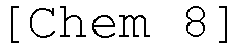 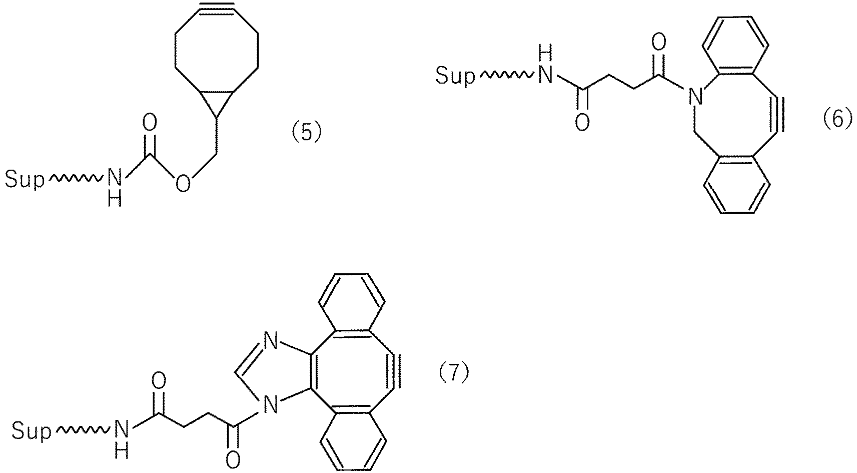 in which Sup is an insoluble support structural moiety.

12. A method for producing a mRNA cap analog, the method comprising the steps of:
introducing into a sugar moiety or a base moiety of a first nucleotide a linker comprising a functional group selected from an amino group, a carboxylic acid active ester group, an aminooxy group, a hydrazino group, a carbonyl group, a thiol group, a haloacetyl group, a Michael acceptor functional group, an alkynyl group, and a 1,3-dipole functional group, the linker having a structure cleavable by light or a fluoride ion; and
linking the first nucleotide having the linker introduced therein to a second nucleotide in the presence of magnesium chloride.

13. A kit for use in production of a mRNA having a 5' cap structure, the kit comprising:
a mRNA cap analogue comprising a linker at a sugar moiety or a base moiety of a nucleotide, the linker having a functional group A selected from an amino group, a carboxylic acid active ester group, an aminooxy group, a hydrazino group, a carbonyl group, a thiol group, a haloacetyl group, a Michael acceptor functional group, an alkynyl group, and a 1,3-dipole functional group, the linker having a structure cleavable by light or a fluoride ion; and
an insoluble support having a functional group B capable of reacting with the functional group A.

14. The kit according to claim 13, wherein the functional groups A and B are a combination of an amino group and a carboxylic acid activated ester, a combination of an aminooxy group or a hydrazino group and a carbonyl group, a combination of a thiol group and a thiol group, a haloacetyl group or a Michael acceptor functional group, or a combination of an alkynyl group and a 1,3-dipole functional group.

15. The kit according to claim 13 or 14, wherein the linker has an o-nitrobenzyl ether structure or a silyl ether structure.

16. The kit according to claim 13, wherein the mRNA cap analog is represented by the following general formula (1) or (2): 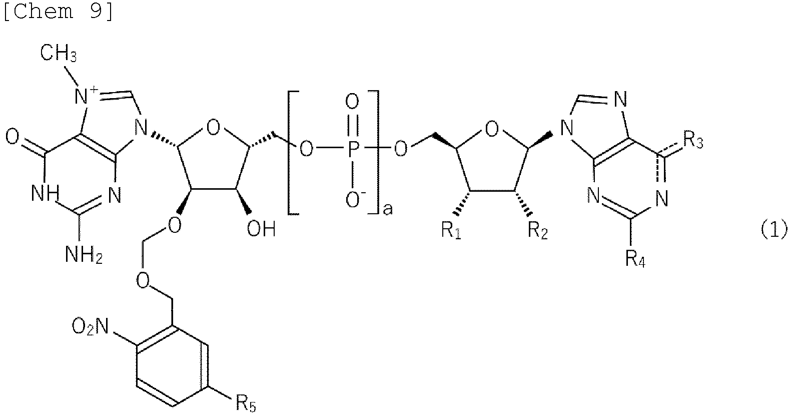 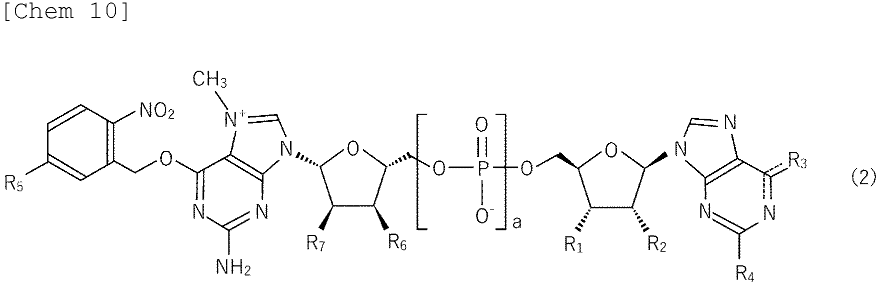 in which:
R₁ is OH, OCH₃ or a group represented by the following general formula (3): 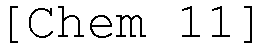 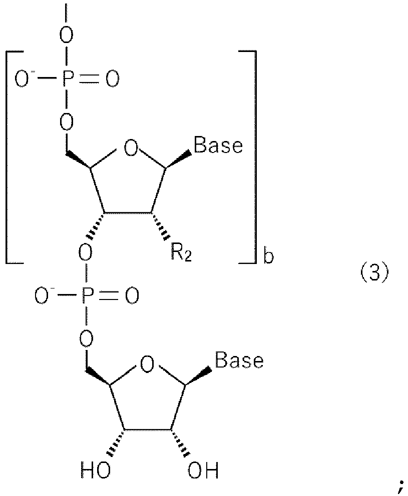
R₂ is OH or OCH₃;
R₃ is O, OH, NH₂, NHCH₃ or N(CH₃)₂, and when R₃ is O, then the bond between the C bonded to R₃ and R₃ is a double bond, and the bond between the C bonded to R₃ and the N adjacent thereto is a single bond, and when R₃ is OH, NH₂, NHCH₃ or N(CH₃)₂, then the bond between the C bonded to R₃ and R₃ is a single bond, and the bond between the C bonded to R₃ and the N adjacent thereto is a double bond;
R₄ is NH₂, NHCH₃, N(CH₃)₂ or H;
R₅ is a propargyloxy group or an azidoethoxy group;
R₆ and R₇, which may be identical or different, are OH or OCH₃;
a is 3 to 6;
b is 0 to 19; and
Base is adenine-9-yl, N6-methyladenine-9-yl, guanine-9-yl, cytosine-1-yl, 5-methylcytosine-1-yl, uracil-1-yl, uracil-5-yl or 1-methyluracil-5-yl.

17. The kit according to claim 16, wherein R₃ is O and R₄ is NH₂.

18. The kit according to claim 16 or 17, wherein, when R₅ is a propargyloxy group, then the insoluble support is represented by the following formula (4): 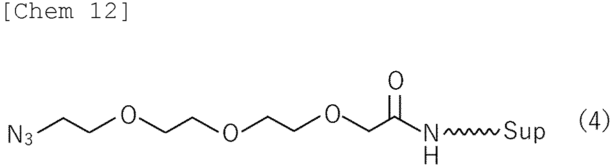 in which Sup is an insoluble support structural moiety.

19. The kit according to claim 16 or 17, wherein, when R₅ is an azidoethoxy group, the insoluble support is represented by the following formula (5), (6) or (7): 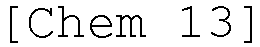 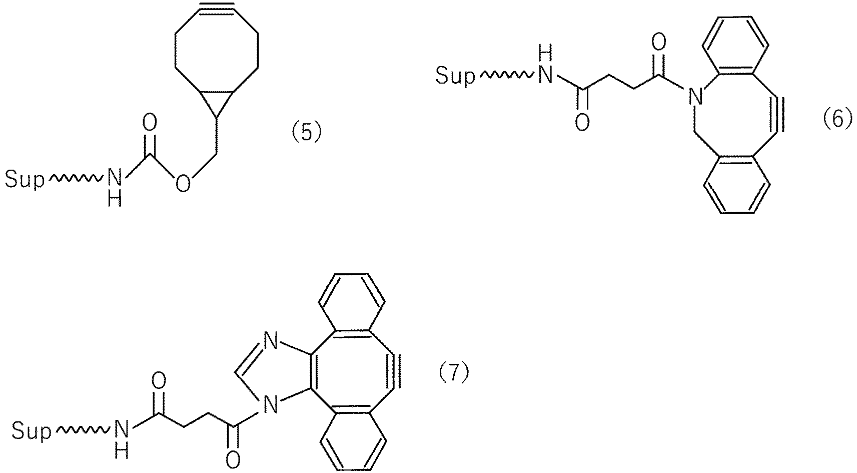 in which Sup is an insoluble support structural moiety.

20. The kit according to any one of claims 13 to 19, further comprising one or more selected from nucleoside triphosphates, template DNAs, and RNA polymerases.
